# EUROPEAN PATENT SPECIFICATION

(11) **EP 4 229 221 B1**
(45) Date of publication and mention of the grant of the patent: **04.03.2026**
(21) Application number: 21790931.6
(22) Date of filing: 19.10.2021
(51) Int. Cl.: C12Q 1/6876, C12Q 1/70

(54) **METHOD FOR PARALLEL NUCLEIC ACID ANALYSIS**
VERFAHREN ZUR PARALLELEN NUKLEINSÄUREANALYSE
PROCÉDÉ D'ANALYSE D'ACIDE NUCLÉIQUE PARALLÈLE

(30) Priority: 19.10.2020 EP 20202627; 05.03.2021 EP 21161020
(43) Date of publication of application: 23.08.2023
(73) Proprietor: IMBA-Institut für Molekulare Biotechnologie GmbH, 1030 Wien (AT)
(72) Inventor: ELLING, Ulrich, 2380 Perchtoldsdorf (AT); YELAGANDULA, Ramesh, 1030 Wien (AT); STARK, Alexander, 1050 Wien (AT); COCHELLA, Maria Luisa, Baltimore, Maryland 21205 (US)
(74) Representative: SONN Patentanwälte GmbH & Co KG
(86) International application number: PCT/EP2021/078911
(87) International publication number: WO 2022/084295

(56) References cited:
- US-A1- 2015 259 734
- US-A1- 2018 355 348
- LAURAE MACCONAILL ET AL: "Unique, dual-indexed sequencing adapters with UMIs effectively eliminate index cross-talk and significantly improve sensitivity of massively parallel sequencing", BMC GENOMICS, BIOMED CENTRAL LTD, LONDON, UK, vol. 19, no. 1, 8 January 2018 (2018-01-08), pages 1 - 10, XP021252292, DOI: 10.1186/S12864-017-4428-5
- DAO THI: "A colorimetric RT-LAMP assay and LAMP-sequencing for detecting SARS-CoV-2 RNA in clinical samples", SCIENCE TRANSLATIONAL MEDICINE, vol. 12, 12 August 2020 (2020-08-12), pages eabc7075 - eabc7088, XP055730132
- JAYAMOHAN HARIKRISHNAN ET AL: "SARS-CoV-2 pandemic: a review of molecular diagnostic tools including sample collection and commercial response with associated advantages and limitations", ANALYTICAL AND BIOANALYTICAL CHEMISTRY, vol. 413, no. 1, 18 October 2020 (2020-10-18), pages 49 - 71, XP037332742, ISSN: 1618-2642, DOI: 10.1007/S00216-020-02958-1
- SIMONE PICELLI ET AL: "Tn5 transposase and tagmentation procedures for massively scaled sequencing projects", GENOME RESEARCH, vol. 24, no. 12, 30 July 2014 (2014-07-30), US, pages 2033 - 2040, XP055236186, ISSN: 1088-9051, DOI: 10.1101/gr.177881.114

## Description

The present invention relates to the field of parallel nucleic acid analytics.

### Background of the invention

Within just a few months the newly emerged coronavirus Sars-CoV2 caused the worldwide pandemic of Covid-19. While the world is awaiting an effective and scalable vaccination and an antiviral therapy, Covid-19 must be contained as good as possible. To this end two measures are available, namely social distancing e.g. via lockdown measures and molecular testing for contact tracing and random surveillance. However, the molecular method to detect active disease, i.e. the presence of viral RNA from swab, gargle, sputum, or saliva, is currently costly and hindered by shortage of equipment and supply of reagents. Large scale testing can be implemented by PCR detection in pooled RNA extracts (Ben-Ami et al., Clinical Microbiology and Infection 2020, 26: 1248-1253). However, such pooling approaches may need retesting to identify the individual positive sample in a positively tested pool. In order to identify individual samples in a multiplex approach, sample-derived DNA is barcoded by a specific nucleotide sequence (Dao Thi et al., Sci. Transl. Med. 2020; 12: eabc7075). The barcode sequence is determined together with the viral sequence, thereby creating the connection of viral presence within the respective sample. In general, however, multiplexing reduces the sensitivity of the assay and/or increases the limit of detection for viral genetic material (Visseaux et al., J Clin Micr 2020, 58(8): e00630-20).

Yet there still remains the need to further increase testing capacity while at the same time reducing testing costs and preferably also maintain or increase test sensitivity. A goal of the present invention is to improve large scale testing capacities to address these needs.

### Summary of the invention

The present invention provided a method for detecting a nucleic acid of interest in a plurality of samples, comprising the steps of
a) providing analyte nucleic acids from a plurality of samples in separate containers for each sample, wherein the containers are arranged into an array of subsets, wherein the array comprises two or more subsets;
b) amplifying nucleic acids by a primer extension reaction using at least one pair of primers hybridized to the analyte nucleic acids, wherein a pair of primers comprises a forward and a reverse primer, wherein the forward and reverse primers both comprise in 5' to 3' direction an adaptor sequence, a sample identifier sequence and a binding sequence for hybridization to the analyte nucleic acids, respectively, and wherein in the forward primer the sample identifier sequence and the binding sequence for hybridization to the analyte nucleic acids are staggered by a random offset of 1-4 bases;
c) combining the amplified nucleic acids of step b) of containers of two or more subsets to an array of combined containers, wherein containers of one subset, but not of another subset, are combined to a combined container;
d) amplifying nucleic acids by a primer extension reaction using at least one pair of further primers hybridized to the amplified nucleic acids of combined containers of step c), wherein a pair of further primers comprises a further forward and a further reverse primer, wherein the further forward and further reverse primers both comprise a subset identifier sequence and a sequence for hybridization to the adaptor sequence;
e) determining the sequences of the amplified nucleic acids of step d);
f) assigning a determined sequence of a nucleic acid of interest of step e) to a sample through association to a subset and container with the subset identifier sequences and the sample identifier sequences.

The invention further provides a set of primers comprising
at least 10 different primers A which comprise the sequence,from 5' to 3': an adaptor A sequence, an identifier sequence of at least 4 nt in length and a target binding sequence, wherein the identifier sequence is different within the at least 10 different primers A, preferably with a sequence distance of a Hamming distance of at least 1 or a Levenshtein distance of at least 1, and wherein the sample identifier sequence and the binding sequence for hybridization to the analyte nucleic acids are staggered by a random offset of 1-4 bases;
at least 10 different primers B which comprise the sequence,from 5' to 3': an adaptor B sequence, an identifier sequence of at least 4 nt in length and a target binding sequence, wherein the identifier sequence is different within the at least 10 different primers B, preferably with a sequence distance of a Hamming distance of at least 1 or a Levenshtein distance of at least 1, and and wherein the sample identifier sequence and the binding sequence for hybridization to the analyte nucleic acids are staggered by a random offset of 1-4 bases;
at least 10 different primers C which comprise the sequence,from 5' to 3': an adaptor C sequence, an identifier sequence of at least 4 nt in length and a binding sequence that binds to the adaptor A sequence, wherein the identifier sequence is different within the at least 10 different primers C, preferably with a sequence distance of a Hamming distance of at least 1 or a Levenshtein distance of at least 1; and
at least 10 different primers D which comprise the sequence,from 5' to 3': an adaptor D sequence, an identifier sequence of at least 4 nt in length and a binding sequence that binds to the adaptor B sequence, wherein the identifier sequence is different within the at least 10 different primers D, preferably with a sequence distance of a Hamming distance of at least 1 or a Levenshtein distance of at least 1.

In preferred embodiments the nucleic acid of interest is RNA, such as from an RNA virus. The method includes a conversion of the RNA to cDNA in step a). As such the inventive method can also be written as a method for detecting a nucleic acid of interest, including ribonucleic acid (RNA), in a plurality of samples, comprising the steps of a) providing analyte nucleic acids from a plurality of samples in separate containers for each sample, wherein the containers are arranged into an array of subsets, wherein the array comprises two or more subsets; comprising reverse transcribing RNA, thereby generating a complementary DNA of the RNA as analyte nucleic acids; b) amplifying nucleic acids, in particular DNA, by a primer extension reaction using at least one pair of primers hybridized to the analyte nucleic acids, wherein a pair of primers comprises a forward and a reverse primer, wherein the forward and reverse primers both comprise an adaptor sequence, a sample identifier sequence and a binding sequence for hybridization to the analyte nucleic acids, respectively; wherein the amplification is preferably DNA specific and/or with a DNA polymerase; c) combining the amplified nucleic acids of step b) of containers of two or more subsets to an array of combined containers, wherein containers of one subset, but not of another subset, are combined to a combined container; d) amplifying nucleic acids by a primer extension reaction using at least one pair of further primers hybridized to the amplified nucleic acids of combined containers of step c), wherein a pair of further primers comprises a further forward and a further reverse primer, wherein the further forward and further reverse primers both comprise a subset identifier sequence and a sequence for hybridization to the adaptor sequence; e) determining the sequences of the amplified nucleic acids of step d); f) assigning a determined sequence of a nucleic acid of interest of step e) to a sample through association to a subset and container with the subset identifier sequences and the sample identifier sequences.

### Detailed description of the invention

The present invention as defined by the appended claims provides a method for detecting a nucleic acid of interest in a plurality of samples. Such a nucleic acid of interest can e.g. be from a pathogen, such as a virus, bacteria or fungi, and thus be used to detected said pathogen via its nucleic acid. This allows the method to be used in the diagnosis of diseases that are associated with the presence of the pathogen.

Usually, the nucleic acid of interest is preselected, i.e. by using primers that bind to the nucleic acid of intertest. As such, the inventive method can be used to detect the presence of specific pathogens of interest.

The inventive method is usually performed in vitro, i.e. a sample is analysed according to the invention ex vivo and/or outside of a subject from whom the sample has been previously obtained.

The subject from whom the sample is obtained can e.g. be a human or a non-human animal, e.g. a mammal, a bird or reptile. Preferably the subject is a mammal, in particular a human.

The sample can be from or contain a biological sample such as a body fluid, such as a sputum, saliva, nasal mucus, bronchoalveolar lavage, urine, a serum or blood sample. Such a sample containing sputum, saliva, or nasal mucus may be obtained from a nasopharyngeal swab, an oropharyngeal swab, a nasal swab, a gargle solution, or providing sputum into a container. More generally the sample may be a solid sample, liquid sample, or aerosols carried in gaseous exhale.

The nucleic acid of interest can be released from the biological sample, cell or encapsulation (e.g. viral encapsulation) so that it is accessible in solution for further treatment. Such a release may include a removal from the pathogenic source, e.g. by the disassembly of the pathogen, which may include virus disassembly or lysis or porosis of bacteria or fungi. Optionally the nucleic acid of interest is stabilized by e.g. removing and/or inactivating any enzymes that would degrade or digest the nucleic acid of interest. E.g. a DNase inhibitor can be added if the nucleic acid of interest is or comprises DNA or a RNase inhibitor can be added if the nucleic acid of interest is or comprises RNA. In combination or as an alternative, the sample may be treated by elevated temperature or chemicals, such as e.g. at an intensity and for a time sufficient to inactivate such unwanted enzymes like DNases and/or RNases.

As said, the nucleic acid of interest can be DNA or RNA, preferably it comprises or consists of RNA.

The nucleic acids of interest, e.g. as released from a biological sample, cell or encapsulation as described above, may be used as the analyte nucleic acids in the inventive methods. In further embodiments, the nucleic acids may be further treated, copied or converted into another type of nucleic acid, such as for example creating a cDNA from an RNA of a biological sample. The cDNA may be generated by reverse transcription. The cDNA may then be used as analyte nucleic acid in the inventive method. A further modification may be fragmentation of a nucleic acid from a sample.

In preferred embodiments of the invention viral RNA is a nucleic acid of interest. Such viral RNA is released from virus capsules, such as capsids or envelopes, and converted into cDNA by performing a reverse transcription. In particular, the invention can comprise in step a) providing RNA from a sample and generating a cDNA of said RNA by reverse transcription, wherein the nucleic acids that are amplified in step b) comprise said cDNA. Reverse transcription can be performed by reverse transcriptase, such as a M-MLV reverse transcriptase, wild type or modified, such as a SuperScript^{™} reverse transcriptase, e.g. SuperScript^{™} III reverse transcriptase or SuperScript^{™} IV reverse transcriptase. Such modified M-MLV reverse transcriptase preferably has reduced RNase H activity and/or increased thermal stability as compared to wild type M-MLV reverse transcriptase under operative conditions (ambient pressure at 1 bar, 50°C). Example primers for reverse transcription can be e.g. random primers, such as random hexamers, which is a collection of various primers with variable sequences at a given length. Example lengths of primers are 5, 6, 7, 8, 9, 10, 11, 12, 13, 14 or more nucleotides in length, preferably 6 (hexamers) or preferably 8-12 for target specific primers. If performed, preferably no barcodes or "identifier sequences" are added during reverse transcription. It is preferred to add such "identifier sequence" in the inventive method later during step b) and d), especially when treating DNA (e.g. cDNA) as analyte nucleic acids. Such barcodes or "identifier sequences" may be later used downstream to connect a determined nucleic acid sequence to a subject from whom the sample originated (and who may thus have an infection with a pathogen).

A container to hold the nucleic acid of a sample may be any holding means suitable for the inventive method. Example containers may be selected from a flask, vial, bag, syringe, or a well, including a microwell on a well-plate, or a droplet. In particular, wells on well-plates are preferred as they allow easy organisation, parallel pipetting, easy handling and automation during the inventive method. Before being put into the preferred container to be used in the inventive method, a sample may have been handled, transported and/or treated in another container, e.g. to facilitate transportation from the patient to the facility performing the inventive method.

Each sample that is distinguished according to the plurality of samples is at least initially in a separate container. Of course, the invention does include using pooled biological samples (e.g. from more than one subject) to be treated as one sample but it is preferred that each sample is associated to only one subject for individual association with the nucleic acid of interest.

The plurality of samples may include 10 or more samples, e.g. 20, 50, 75, 100, 150, 200, 250, 300, 400, 500, 600, 700, 800, 900, 1000, 1200, 1400, 1600, 1800, 2000, 2500, 3000, 4000, 5000, 6000, 7000, 8000, 9000, 9500, 10000, 12000, or more samples and any range in between these values. For example, the plurality of samples is 20 to 1000000 samples, e.g. 40 to 500000 samples or 90 to 100000 samples.

In step a) of the inventive method, analyte nucleic acids from a plurality of samples are provided in separate containers for each sample. The analyte nucleic acids may be RNA or DNA; DNA is preferred, as such the analyte nucleic acids may comprises or consists of DNA. If the nucleic acid of interest, e.g. from a pathogen, is RNA, it is preferred to generate a cDNA therefrom to be used as the analyte nucleic acid, as discussed above. The generation of cDNA may be done in the same containers that are later used during the inventive method. Enzymes, like a reverse transcriptase may be inactivated, such as by elevated temperature (e.g. 70°C or more for a SuperScript^{™} reverse transcriptase), before continuing with step b).

The containers are arranged into an array of subsets, wherein the array comprises two or more subsets. "array of subsets" means that the containers (with the analyte nucleic acids) are grouped in at least two groups ("array comprises two or more subsets"). This arrangement can be an information available to the user who performs the inventive method and/or a special grouping from which the subset arrangement is apparent and/or a labelling or marking of the containers. For example, the containers may be arranged into an array of subsets and the subsets comprise the containers at a coordinate. That coordinate may be noted and information of its container may be stored by the user. Coordinates may be of a 2D or 3D arrangement, e.g. rows, columns and/or stacks of one or more carrier(s) with the containers. Such a carrier may be a plate, e.g. with depressions as containers, such as a well plate. Of course, any other form of coordinate may be used to identify a container of a subset and/or of a sample from a particular subject, including water-based droplets in oil. Containers may have walls or not, such as in the case of droplets. In a preferred embodiment, the containers are arranged into an array of subsets and the subsets comprise the containers at a coordinate. The containers with a corresponding coordinate for each subset are combined in step c).

An example is using two or more well-plates. All wells (containers) in one well-plate are a subset with each well-plate forming a subset of its wells (containers). According to this example, a plurality of two or more well-plates would thus contain an array of two or more subsets with the subset amount being the number of well-plates and each subset (individual well-plate) consisting of the number of wells (containers) per plate or less, if not all wells contain an analyte nucleic acid. In fact, in preferred embodiments such a well-plate (subset) may use 1-10 of its wells (containers) for controls, so that in case of 96-well plates fewer than 96 wells may be used for analyte nucleic acids. Irrespective of this example, in any embodiment of the invention, each subset may comprise one or more control nucleic acids or empty controls without nucleic acids in a container, that are treated similarly throughout the inventive method as containers with analyte nucleic acids.

In preferred embodiments of the invention, the two or more subsets are 8 or more subsets, 10 or more subsets, such as 20 or more subsets, e.g. 30 or more subsets, 40 or more subsets, 50 or more subsets, 60 or more subsets, 70 or more subsets, 80 or more subsets, or 90 or more subsets. An example for 8 subsets are 8 well plates of PCR strips.

In equally preferred embodiments, a subset comprises 10 or more containers, preferably 20 or more containers, or 30 or more containers, or 40 or more containers, or 50 or more containers, or 60 or more containers, or 70 or more containers, or 80 or more containers, or 90 or more containers.

Examples of such number for subset or container arrays are well plates such as 96-, 384- or 1536-well plates. Any such number as mentioned above (at least 2, 10, 20, etc.) of wells can be used as containers or members of subsets of the present invention. Remaining wells on a plate may or may not be used, e.g. as controls. With the subset representing plates or in general any such array, the number of subsets represents the number of plates or arrays. When subsets are combined in step c), their combined nucleic acids may also be stored in a container, with each combined nucleic acids of a subset being in a separate container. Again, in some examples, well plates may be used, with e.g. one or more wells containing the combined nucleic acids of a subset. In preferred embodiments, the method uses 96 containers (subset size) and 96 subsets; in further options the method uses 384 containers (subset size) and 96 or 384 subsets. This would make full use of 96- or 384 well plates. Also 1536 well plates can be used for the containers or the subsets. In any of these embodiments, the number of containers to accommodate samples or subsets (when combined in the step d) can be lower to accommodate controls as mentioned above.

In step b) of the inventive method, nucleic acids are amplified by a primer extension reaction using at least one pair of primers hybridized to the analyte nucleic acids, wherein a pair of primers comprises a forward and a reverse primer, wherein the forward and reverse primers both comprise an adaptor sequence, a sample identifier sequence ("sample index") and a binding sequence for hybridization to the analyte nucleic acids, respectively.

As mentioned above, the analyte nucleic acids are preferably DNA - either because DNA has been provided by the sample or after conversion of RNA into cDNA with the cDNA being used as analyte nucleic acids in step b). Primer extension reactions are conventional in the art and include binding the primers to the analyte nucleic acids and extending the primers with further nucleotides in a template specific fashion, with the template being the analyte nucleic acid. Accordingly, a complementary strand is generated if the sample contains the appropriate template. Such a reaction is preferably performed with a DNA polymerase. A second strand can be generated likewise with another primer. Both primers are referred to as forward and reverse primers for first and second strand generation as is common in the art. An example of a primer extension reaction is PCR.

Alternatively, the analyte nucleic acid is RNA. With RNA as template, it is preferred to generate a complementary DNA strand (also termed copy DNA or cDNA). As such the (forward) primer preferably is DNA with the extended nucleotides also being DNA. Such a reaction is for example a reverse transcription, which can be facilitated by a reverse transcriptase. Second strand generation with the reverse primer is then a DNA templated DNA polymerisation, preferably with a DNA polymerase as described above. Further rounds or cycles of amplification can then be entirely DNA-based, including primer extension reactions using the forward primer, which in a further round or cycle may use the generated complementary strand of the reverse primers' amplification product as template. The difference with the previous paragraph - according to this embodiment - would be that in the previous paragraph, both forward and reverse primers add their sample identifier in entirely DNA-based amplification procedures, whereas the second alternative adds primers with the sample identifier already in a reverse transcription reaction (or generally in an RNA-based amplification reaction). It is preferred according to the invention to add the sample identifier only using DNA templates since DNA-based primer extension reactions are more robust with fewer side reactions but of course using RNA templates is also a possibility, in particular given that the invention uses two sample identifiers (on both, the forward and reverse primers) to compensate and even add further reliability to the method, thereby increasing specificity.

As said, according to the invention, the forward and reverse primers both comprise an adaptor sequence, a sample identifier sequence and a binding sequence for hybridization to the analyte nucleic acids, respectively.

The binding sequence for hybridization to the analyte nucleic acids comprises a complementary sequence to the analyte nucleic acid (or its complementary sequence in case of the reverse primer) so that the primer specifically binds to the analyte nucleic acid (or its complementary sequence in case of the reverse primer) by hybridisation under reaction conditions. Reaction conditions for hybridization may vary according to the enzymes and/or buffers used and may e.g. comprise heating to 58°C in a PCR buffer comprising 750 mM Tris-HCl pH 8.3, 200 mM (NH₄)₂SO₄, 1% Triton X-100, which is a preferred embodiment of the invention. Also variations of the buffers can be used. E.g. a buffer may comprise a pH of 7 to 9, preferably about 8.3. Preferably sulphate ions are present; also preferred a crowding agent such as Triton X-100 is used; preferably in a concentration of 0.3 to 3%, e.g. about 1%, (all w/w-%). Preferably the complementary sequence to the analyte nucleic acid comprises 8 or more, e.g. 9, 10, 11, 12, 13, 14, 15, 16, 17, 18, 19, 20, 21, 22 or even more nucleotides, e.g. 8-30 nucleotides that are complementary with the analyte nucleic acid (or its complementary sequence in case of the reverse primer). The number of complementary nucleotides of the forward and reverse primer can be selected independently. Through the binding sequence, the primers are specific to an analyte nucleic acid sequence of interest. These analyte sequences can e.g. be from a pathogen as will be elaborated in more detail below.

The adaptor sequences on the forward and reverse primers are sequences that are used downstream of the inventive method to bind further forward and reverse primers in step d) to the amplified nucleic acids given that the adaptor sequences are preserved in the amplification products. Such adaptor sequences are usually artificial and usually do not bind to expected nucleic acids in the sample. Suitable adaptor sequences are known in the art and have e.g. been deposited in databases (e.g. the Illumina Adapter Sequences 1000000002694 v14 of July 2020). Any such adaptors can be used according to the invention. Generally, almost any sequence is possible, but of course complementary sequences are used in the further forward and reverse primers of step d), which thus form interrelated primers with the forward and reverse primers of step b). Of course, only one primer sequence in the amplified nucleic acids is bound by each sequence for hybridization to the adaptor sequence in the further forward and reverse primers. For example, the sequence for hybridization to the adaptor sequence in the further forward primer binds to the adaptor sequence of the forward primer but not of the reverse primer; the sequence for hybridization to the adaptor sequence of the further reverse primer binds to the adaptor sequence of the reverse primer but not of the forward primer; or both vice-versa. The adaptor sequence of the forward and reverse primer, selected independently, can be 8 or more, e.g. 9, 10, 11, 12, 13, 14, 15, 16, 17, 18, 19, 20, 21, 22 or even more nucleotides, e.g. 8-30 nucleotides in length.

The sample identifier sequence is a label or barcode that identifies the amplification product of a sample and is introduced in said amplification product via the primers. The sample identifier sequences are usually selected to identify a sample within a subset. Since further identifier sequences are introduced in step d), it is not needed to uniquely label each sample of the entire array. The sample identifier sequences can be shared between different types of analyte nucleic acids/binding sequence for hybridization to the analyte nucleic acids. Nucleic acids of different subsets may be labelled with sample identifier sequences that are selected from the same pool of sample identifier sequences and/or may comprise nucleic acids that are labelled by the same sample identifier sequences. Since both the forward and reverse primers comprise sample identifier sequences - selected independently - the inventive method introduces high specificity. In a preferred embodiment, the sample identifier sequences of the forward and reverse primers of a given container/sample should not be identical or at least not selected to be the same with identities being only random occurrences that should be below 5% or below 1% of the containers or samples. The inventors have noticed with this method that the incidence of false positive results can be significantly reduced, which otherwise occurs when high numbers of amplification cycles are used. Unwanted amplification reactions may lead to a product despite the absence of the nucleic acid of interest in a sample, thus leading to a false positive result - if no further control steps are incurred.

Preferably in step b) each sample identifier sequence of the forward primers is different from the sample identifier sequences of other forward primers for a given binding sequence for hybridization to the analyte nucleic acids. In other words, the sample identifier sequences are each unique for a given analyte nucleic acid or binding sequence for hybridization to the analyte nucleic acid - or in the final amplification product - unique for a given amplicon. For different binding sequences for hybridization to the analyte nucleic acid/amplicons the same sample identifier sequences may be used. In similar preferred embodiments, in step b) each sample identifier sequence of the reverse primers is different from the sample identifier sequences of other reverse primers for a given binding sequence for hybridization to the analyte nucleic acids. These two preferred embodiments can of course be combined; i.e. the sample identifier sequences of the forward and reverse primers are unique for a given binding sequences for hybridization to the analyte nucleic acid/amplicons. In other embodiments, the sample identifier sequences may also be unique among the combined group of forward and reverse primers, but this is not required but of course optional - optionally only for a given binding sequences for hybridization to the analyte nucleic acid/amplicon or not only for a given binding sequences for hybridization to the analyte nucleic acid/amplicon.

The inventive method is based on repeated amplifications of analyte nucleic acids and then of complementary nucleic acids ("amplification cycles"). Each amplification initiated by at least either a forward or reverse primer is considered an amplification cycle. Thus, for illustration, an amplification reaction of both the forward and reverse primer but no further amplification reaction is considered as a reaction with 2 amplification cycles. 2 amplification cycles are thus the minimum for both the forward and reverse primer to get incorporated into the amplification product, for labelling with the sample identifiers. If both forward and reverse primers are present as well as templates for both the forward and the reverse primer, then both primers will result in an amplification product in one amplification cycle. This is usually the case for amplification cycle number 2 onwards. In experimental practice, an amplification cycle is one round of heating and cooling. High numbers of amplification cycles increase the number of erroneous results. However, large numbers of amplification cycles may be needed to detect a nucleic acid of interest or analyte nucleic acid that is present in low amounts. Standardized conditions for all samples/containers are usually used, i.e. the same number of amplification cycles for all containers/samples. In some containers, high numbers of analyte nucleic acids may be present, whereas in other containers low numbers are present. Prior to the invention, this caused sub-optimal conditions for some samples leading to false positive or false negative results. The present invention has improved on such methods to allow sufficient sensitivity and selectivity to detect various samples in one multiplexed reaction, as e.g. shown in the examples. The invention works with various amplification cycles for step b), such as 2-80 amplification cycles, preferably 5 to 75, 10 to 70, 20 to 65, 25 to 60, 30 to 55, 35 to 50, or 40 to 45 amplification cycles, or any ranges between these values. In preferred embodiments the amplification of step b) is an endpoint amplification. According to this embodiment that is preferred in all aspects of the invention as it strives towards equalizing the number of amplified nucleic acids in each container regardless of initial nucleic acid amount. This greatly helps in detecting nucleic acids of interest at various concentration and amount ranges in the inventive parallel detection method. Endpoint amplification means that the amplification reaction is carried out until no further amplification can occur (in each container). This can be caused by the fact that the required nucleotides for a further amplification cycle are consumed and/or the primers for another amplification cycle have been consumed. A container with fewer nucleic acids that are amplified will take more amplification cycles than a container with a higher number of nucleic acids that are amplified. Accordingly, since all containers are treated similarly, i.e. with the same number of amplification cycles, a number of amplification cycles is selected that will amplify a container with a minimum amount of nucleic acids of interest that are to be detected. Of course, an empty container will not produce any amplification products regardless of the number of amplification cycles and amounts of nucleotides and/or primers. Primer and/or nucleotide consumption during amplification can be due to analyte nucleic acids (if present), controls and/or spike-ins. If no controls and spike-ins are present, then a detection limit may be chosen for a minimum number of analyte nucleic acids that lead in a given number of cycles to the endpoint (no further complete amplification possible). Such a minimum number of analyte nucleic acids can be e.g. 10 or 100 or 500 or 1000 analyte nucleic acids. The selected number of amplification cycles shall take into account the amounts of nucleotides and primers that are added, which determine the cycle number until they are consumed (amplification to saturation). Accordingly, in preferred embodiments of the invention, the number of amplification cycles in step b) is selected so that no further amplification of an analyte nucleic acid occurs in the containers. Under normal circumstances 40 or more, e.g. 45 or more cycles are sufficient for an endpoint amplification. A skilled practitioner can choose a suitable amplification cycle number for the sample at hand. In general, if low amounts of nucleic acids of interest are expected to be in a sample, then higher numbers of amplification cycles will be selected. With the present invention, it was possible to detect nucleic acids of interest that had high differences in sample abundance, with samples differing by a factor of up to x10⁷ copies of nucleic acids of interest per sample. It was quite surprising that such differing samples could be processed in one multiplexed reaction at the same conditions for all samples.

In a preferred structure of the forward and/or the reverse primer the binding sequence for hybridization to the analyte nucleic acids is preferably on the 3' end of the forward and/or reverse primer. This allows an efficient extension reaction in 5' to 3' direction. Preferably the sample identifier sequence is between the binding sequence and the adaptor sequence. The adaptor sequence may be at or near the 5' end of the forward and/or reverse primer. Accordingly, a preferred primer structure for the forward and/or reverse primer comprises in 5' to 3' direction: [adaptor sequence] - [sample identifier sequence] - [binding sequence].

Step c) comprises combining the amplified nucleic acids of step b) of containers of two or more subsets to an array of combined containers, wherein containers of one subset, but not of another subset, are combined to a combined container. Accordingly, the containers of one subset are combined to the combined container. This is done for more than two subsets, thereby creating two or more combined containers, each corresponding to one subset. Given that the analyte nucleic acids have been amplified to create amplification products that are labelled by the sample identifier in step b), each amplified nucleic acid carries the sample/original container information despite the combination into one pool of multiple different amplification products of different analyte nucleic acids in the combined container.

In preferred embodiments of the invention, the two or more subsets that are combined (for each subset separately of course) are preferably 10 or more subsets, such as 20 or more subsets, e.g. 30 or more subsets, 40 or more subsets, 50 or more subsets, 60 or more subsets, 70 or more subsets, 80 or more subsets, or 90 or more subsets. In preferred embodiments 96 subsets, 384 or 1536 subsets are combined to fully use 96-, 384-, or 1536-well plates with each well containing a combined subset. The number of containers to accommodate subsets can be lower to accommodate controls as mentioned above.

The combined containers may be physically similar to the containers described above, but of course contain various amplified nucleic acids. Example combined containers may be selected from a flask, vial, bag, syringe, or a well, including a microwell on a well-plate. In particular, a well-plate is preferred with different wells being or containing different combined containers.

Step d) comprises amplifying nucleic acids by a primer extension reaction using at least one pair of further primers hybridized to the amplified nucleic acids of combined containers of step c), wherein a pair of further primers comprises a further forward and a further reverse primer, wherein the further forward and further reverse primers both comprise a subset identifier sequence ("subset index") and a sequence for hybridization to the adaptor sequence. Step d) is similar to step b), wherein now amplification products are amplified instead of analyte nucleic acids, the primers are referred to as "further primers", and the label is now used to identify the subset (or combined container) instead of the analyte. Adaptors for a further amplification reaction or other uses, such as a sequencing adaptor sequence, are now optional and may or may not be present. The further primers bind to the adaptor sequences that have been incorporated into the amplification products through the primers of step b) as described above.

In a preferred structure of the further forward and/or the reverse primer the sequence for hybridization to the adaptor sequence is preferably on the 3' end of the forward and/or reverse primer. This allows an efficient extension reaction in 5' to 3' direction. Preferably the subset identifier sequence is in 5' direction from the sequence for hybridization to the adaptor sequence.

In preferred embodiments, in step d) each subset identifier sequence of the further forward primers is different from subset identifier sequence of other further forward primers; and/or in step d) each subset identifier sequence of the further reverse primers is different from subset identifier sequence of other further reverse primers. As said above with regard to the uniqueness of the forward and reverse primers in step b), also the further forward and/or reverse primers are unique among each group, i.e. subset identifier sequences of further forward primers differ from other subset identifier sequences of other further forward primers. The same applies for the subset identifier sequence of further reverse primers. Accordingly, in this preferred embodiment the subset identifier sequences are unique within the respective groups of further forward primers and the further reverse primers. In other embodiments, subset identifier sequences may also be unique among the combined group of further forward and further reverse primers, but this is not required but of course optional.

In preferred embodiments, the further forward and/or the reverse primer may comprise a further adaptor sequence. Such a further adapter sequence may allow further primer binding reaction or binding to probes during a sequencing reaction, as such the further primer is also referred to as a "sequencing adaptor sequence". When such a further adaptor is present, it is preferred that the subset identifier sequence is between the sequence for hybridization to the adaptor sequence and the sequencing or further adaptor sequence. The further or sequencing adaptor sequence may be at or near the 5' end of the further forward and/or reverse primer. Accordingly, a preferred primer structure for the further forward and/or reverse primer comprises in 5' to 3' direction: [sequencing adaptor sequence] - [subset identifier sequence] - [sequence for hybridization to the adaptor sequence].

In preferred embodiments, the main amplification leading to a substantial increase in amplification products is in step b). Further amplification can be achieved in step d). However, it is preferred to leave the amplification focus on step b). Accordingly, in this preferred embodiment, only few amplification cycles are done in step d). At least two amplification cycles should be done to have a reaction of both the further forward primer and of the further reverse primer. The invention works with few but also several amplification cycles for step d), such as up to 35 amplification cycles or up to 20 amplification cycles. In preferred embodiments 2 to 14 amplification cycles, preferably 3 to 13, 4 to 12, 5 to 11, 6 to 10, 7 to 9, or 8 amplification cycles, or any ranges between these values are used. In preferred embodiments, amplifying nucleic acids in step d) is restricted to at most 12 amplification cycles, preferably at most 10 amplification cycles.

In preferred embodiments, the amplification product as obtained in step b) is DNA. Also in step d), preferably DNA amplification products are obtained. A DNA polymerase can be used as described above for step b). Likewise, also a PCR reaction is preferred for step d).

In further preferred embodiments, in step d) staggered primers are used to amplify nucleic acids. "Staggered primers" means that multiple primers are used, wherein the primers have 0 to 10, such as 1, 2, 3, 4, 5, 6, 7, 8, 9, or 10, preferably 1 to 4, inserted nucleotides as distancing nucleotides between the "further adaptor sequence" (e.g. "sequencing adaptor") and the subset identifier sequence. This embodiment applies to both or one of the further forward and/or further reverse primers. Usually only one of the further primers, either the further forward or further reverse primer, has these distancing nucleotides to have their benefits, when sequencing of amplification products is from one end of the amplification products. Preferably the further forward primers comprise the distancing nucleotides. The distancing nucleotides cause an offset during sequencing of different amplified nucleic acids in step e) so that a specific nucleotide of the subset identifier sequence is at a different distance to the further adaptor sequence and is thus read at a different time-point or instance of the sequencing reaction. The distancing nucleates are preferably random, i.e. mixtures of A, G, C, T at a given position in the primer. Having various distances reduces competitive reactions and/or optimizes the gain of the sequencer when sequencing the same nucleotide position, which without the staggering would be the same nucleotide type (e.g. G, A, C, T) at the same time or instance. A distancing nucleotide can be any nucleotide type, preferably at a given position (distance from further adaptor sequence) different distancing nucleotides are used in different primers to avoid creating such competitive reactions with the distancing nucleotides. Such staggered primers (mixtures of primers with different distancing nucleotides) are used per container/subset. A particular container may have a constant number of distancing nucleotides, whereas other containers have other numbers of distancing nucleotides.

Step e) comprises determining the sequences of the amplified nucleic acids of step d). Sequence determination can be done in the way as known in the art, e.g. by Sanger sequencing or next generation sequencing. Preferably, for sequencing, the amplified nucleic acids of step d) are immobilized on a solid phase, such as on a column or on beads. An example is flow cell sequencing. For such immobilization binding to probes on the solid phase is possible, e.g. by the sequencing adaptors mentioned above. Sequencing adaptors may be Illumina P5 and/or P7 adaptors. Either only one end of the amplified nucleic acids of step d) is immobilized or both ends of the amplified nucleic acids of step d) are immobilized, thereby forming a loop with the solid phase. For one end immobilization only one of the further forward primer and further reverse primers may have a sequencing adaptor; for both end immobilization both of the further forward primer and further reverse primers may have a sequencing adaptor.

The sequences that are determined in step e) are the subset identifier sequences as introduced by the further forward and further reverse primers into the amplified nucleic acids, the sample identifier sequences as introduced by the forward and reverse primers into the amplified nucleic acids and the analyte sequence that corresponds to the sequence between the region that is hybridized in the analyte nucleic acid by the binding sequences for hybridization to the analyte nucleic acids of the forward and reverse primers. The analyte sequence corresponds to and identifies the nucleic acid of interest and the sample identifier sequences and the subset identifier sequences correspond to and identify a sample.

Preferably the binding sequences for hybridization to the analyte nucleic acids in the forward and reverse primers are selected so that costs for sequencing are limited, i.e. the region between these corresponding regions on the analyte molecule - leading to the analyte sequence (amplicon) - has a length of at least 10 and/or up to 400 nucleotides, preferably 20 to 300 nucleotides, even more preferred 30 to 200 nucleotides, such as 40 to 150 nucleotides, even more preferred 45 to 100 nucleotides, or about 70 nucleotides.

In preferred embodiments, any amplicons for analyte nucleic acids, spike-ins, internal and added controls are all within these ranges, in particular preferred are approximately the same, such as within 30% of their length (+/-30% of the longest amplicon length in nucleotides). Having similar lengths minimized competitive reactions.

As a data processing step that follows from the information generated in step e), in step f) a determined sequence of a nucleic acid of interest of step e) is assigned to a sample through association to a subset and container with the subset identifier sequences and the sample identifier sequences. In other embodiments, step f) can be performed with technical means, such as on a computer. The computer may receive sample identification information, such as the location of its container, e.g. in the array, the subset identifier sequences and the sample identifier sequences and the determined sequences and present the information of a detected nucleic acid of interest, via its analyte sequence, in a sample. Preferably the information given in step f) includes a yes or no information of the nucleotide acid of interest being or not being in a sample. The computer, or the inventive set or kit, may comprise a computer readable data memory device, such as a flash memory or a hard disk, to store instructions for performing such a method. In some embodiments of the invention, step f) may be omitted or outsourced to another service provider, who is not performing the method of steps a) to e).

In preferred embodiments of the inventive method, at least one of the forward and reverse primers of step b) is depleted during and/or after step b) but before step c). Alternatively or in combination, preferably in combination also free nucleotides (substrate in an amplification reaction) are depleted. Such a depletion reduces the risk of adverse reaction based on priming by the forward and reverse primers during step d). Such a depletion can e.g. be a physical removal, such as by binding to a solid phase, such as beads and removing the beads with the primers. The solid phase may comprise complementary sequences that bind single stranded primers but not double stranded amplified products. Preferably the primers and/or nucleotides are enzymatically depleted, e.g. by a nuclease or phosphatase, which removes phosphates from primers and/or nucleotides 3' end so that they are unsuitable for further extension reactions. An example is the enzyme mixture ExoStar^{™}. Preferably, exonuclease 1 and/or alkaline phosphatase are used for enzymatic depletion of primers and/or nucleotides.

In preferred embodiments of the invention with a reverse transcription of RNA nucleic acids of interest in step a) as mentioned above, it is also preferred to deplete primers (RT primers) and/or nucleotides before step b) in order to avoid adverse reactions. Said depletion can be performed mechanically or enzymatically as described above, e.g. by purification, absorption, e.g. on a solid phase, or enzymatic activity, such as by a phosphatase or nuclease.

Another method of depletion would be amplification of a spike-in nucleic acid with the forward and/or reverse primer. In this embodiment, the forward and reverse primers bind to the spike-in nucleic acid (or just "spike-in"), that has a complementary nucleic acid sequence to the binding sequence for hybridization to the analyte nucleic acids of the primers but a different sequence than the analyte nucleic acids between said complementary sequences so as to not confuse the spike-ins with nucleic acids of interest. The spike-in nucleic acids thus create a competitive amplification reaction to the analyte nucleic acids and thereby deplete the primers. Since such a competitive reaction may reduce sensitivity of analyte nucleic acids, it is preferred to deplete the primers, e.g. physically or enzymatically. However, it was surprisingly found that in most instances of the inventive method such a competitive reaction does not hinder the detection of analyte nucleic acids - see examples.

An enzymatic removal can e.g. be with a single strand specific nuclease. This is one of the preferred embodiments. Thus, the inventive method may comprise a depletion of the forward and reverse primers, comprising treatment with a single strand specific nuclease.

Spike-ins or any other added control nucleic acids, that is/are added to a container and are amplified and labelled like an analyte nucleic acid in the inventive method, are used in preferred embodiments for all containers. Such spike-ins and controls can be used to identify problems in the inventive detection method, e.g. if an inhibitor of any reaction was present. Spike-ins and added control nucleic acids should produce a detectable product at step e) regardless of sample quality.

The inventive method may further comprise amplifying and processing through steps a) to d), preferably also steps e) and or f), an internal control of the sample, which is a nucleic acid or gene or its coding region that is expected to exist in a sample regardless of the nucleic acid of interest. Such internal controls may e.g. be from constitutively expressed genes of a subject, e.g. a human. In preferred embodiments, the internal control may be a ribosomal gene or coding sequence. A primer pair that binds to a ribosomal gene or coding sequence or any other internal control of a nucleic acid that is expected to exist in a sample may be used in step a) (in case of RNA) or step b) (in particular in case of DNA). RNA internal controls are preferred. A ribosomal gene or coding region or other internal control may be amplified in parallel to the analyte nucleic acids and its amplification products in step b) and d) as a control. The control reaction may be contained in a separate container without analyte nucleic acids or in a container with analyte nucleic acids.

In particular the comparison of spike-ins' and added controls' products of step d) can be compared with step d)'s products of internal controls of a sample. This comparison can be an indicator of problems in the inventive method (when the added controls do not produce an expected product or amount thereof) or provide an indicator if the sample was not isolated or processed properly (if the added control or spike-in produces an expected product in step d) but not the internal control). Such problems can e.g. be improper gargling and other improper sample procurement methods.

The nucleic acid of interest can be a pathogen nucleic acid. Such a pathogen nucleic acid may be characteristic of a particular pathogen. By identifying the pathogen nucleic acid, the presence of at least parts of a pathogen in the sample can be inferred. Preferably the pathogen nucleic acid is a viral nucleic acid.

In general, the nucleic acid of interest or analyte sequence can be from any organism, including a virus, a bacterium, or fungi. An example are gut bacteria. A characteristic sequence in any organism can be detected, in particular identified, with the inventive method.

In a particular preferred embodiment of the invention, the nucleic acid of interest is selected from a SARS-CoV-2 nucleic acid, an influenza nucleic acid, a parainfluenza nucleic acid, a respiratory syncytial virus nucleic acid, a rhinovirus nucleic acid, or a combination thereof. When referring to combinations of the nucleic acid of interest two or more nucleic acids of interest are detected in parallel in the inventive method. Accordingly, more primer pairs are included in step b) that are specific for the analyte nucleic acids corresponding to the two or more nucleic acids of interest, via their binding sequence for hybridization to the analyte nucleic acids. Also, pathogen specific primers may optionally be used in step a), in a reverse transcription, or in step b) to increase sensitivity of the method.

In preferred embodiments of the invention two or more nucleic acids of interest are selected for detection in the inventive method, preferably wherein the two or more nucleic acids of interest are from a virus. The virus can be the same virus or a different virus for two or more nucleic acids of interest. In preferred embodiments in step b) forward and reverse primers with binding sequences for hybridization to the two or more analyte nucleic acids are used. The two or more nucleic acids of interest can be searched for in step f) and assigned to a sample.

In preferred embodiments, 1, 2, 3, 4, 5, 6, 7, 8 or more nucleic acids of interest, preferably two or more nucleic acids of interest, are detected in parallel. In preferred embodiments the two or more nucleic acids of interest are detected from 1, 2, 3, 4, 5, 6, 7, 8 or more organisms, such as pathogens. E.g. it is possible to detect two or more different nucleic acids of interest from the same organism, such as a pathogen, or from differing organisms, e.g. pathogens. In preferred embodiments, the choice of amplicons also includes an internal control directed towards host RNA as sample quality control.

The pathogen may be a virus, a bacterium, or a fungal pathogen.

Example viruses include enterovirus, metapneumovirus, adenovirus, influenza A, B, C, D virus, respiratory syncytial virus (RSV), SARS-associated coronavirus, including SARS-CoV-1, SARS-CoV-2, MERS-CoV, rubeola virus, varicella zoster virus, norovirus, rotavirus, enterovirus, among others. Preferably the virus is an RNA virus.

In preferred embodiments, the present invention can be employed to detect variants of one or more pathogens or variants of one or more marker proteins, e.g. those variants that are indicative for a pathogen's pathogenicity and/or its reactivity to a hosts immune system, or the progression, severability and/or treatability of a disease (e.g. infection, or tumour).

In preferred embodiments, the one or more pathogens are viruses. In particular preferred embodiments the virus to be detected includes a particular variant or strain of SARS-CoV-2, such as for example 20A.EU1, 20A.EU2, B.1.1.7 (also known as 20I/501Y.V1 or Alpha), B.1.351 (also known as 20H/501Y.V2 or Beta), P.1 (also known as 20J/501Y.V3 or Gamma), B.1.617.2 and AY lineages (also known as Delta), B.1.525 (also known as Eta), B.1.526 (also known as Iota), B.1.617.1 (also known as Kappa), B.1.617.3, P.2 (also known as Zeta), B.1.621 (also known as Mu), 20B/S.484K, as well as variants or strains with mutations or deletions in the Spike protein, including S:S13I, S:L18F, S:D80Y, S:S98Y, S:D138Y, S:W152C, S:L189F, S:P209H, S:A222V, S:P272L, S:K417N, S:N439K, S:K444N, S:452R, S:S477N, S:T478K, S:N501Y, S:D614G, S:Q677H, S:A222V, S:S477N, S:N501, especially S:N501Y, S:N501T, S:N501S, S:E484, especially S:E484K, S:N453F, S:S98F, S:L452R, S:D80Y, S:A522S, S:E583D, S:A626S, S:Q675R, S:P681, especially S:P681H, S:P681R, S:P681L, S:I692V, S:V772I, S:V1122L, S:M1229I, S:A570D, S:D614G, S:P681H, S:T716I, S:S982A, S:D1118H, and deletion variant with deletion S:H69-, S:V70-, S:69/70-, S:144-, or variants with mutations at other loci, such as ORF10:V30L, N: S186Y, N: D377Y, N:P199L, N:A220V, N:M234I, N:A376T, ORF1b:A176S, ORF1b:V767L, ORF1b:K1141R, ORF1b:E1184D, ORF1a:I2501T, ORF3a:Q38R, ORF3a:G172R, ORF3a:V202L, ORF1a:I4205V, ORF1b:D1183Y, ORF1a:T945I, ORF1a:T1567I, ORF1a:Q3346K, ORF1a:V3475F, ORF1a:M3862I, ORF1b:P255T, ORF7a:R80I, or combinations thereof, such as in B.1.1.7, e.g. a combination of S:deletion 69-70, S:deletion 144, S:N501Y, S:A570D, S:D614G, S:P681H, S:T716I, S:S982A, S:D1118H.

The nucleic acid of interest to be detected may comprise a nucleic acid of the SARS-CoV-2 spike protein. The spike protein is highly indicative of SARS-CoV-2 infections and the virus' virality or reactivity to a host's immune system as well as its capability to dock to its cellular receptor, ACE2.

Example variants of the spike protein that may be detected with the inventive method may comprise any one of the following mutations: any one or more mutations selected from E484Q E484G K417N F456V T478I E484A S494Q N439K F490S S477R S477I S477N N501T K417T T478R L455F N501Y G446S E484K Y449N T478K S494P (in GH clade (B.l.*); any one or more mutations selected from E484Q T478I N439K Y449H G446V F490S Y495H F490L S477N Y489H N501T G476S K417T G496S L455F N501Y V445I E484K T478K G485R S494P (in GR & GRY clade, e.g. B.l.1.1 & B.1.1.7); any one or more mutations selected from E484Q T478I A475T S494T F490I G504S S477N G476S S494A N501Y G446L G447V V503A P499R E484K E484V Q493L K458N T500S Y453H Y505W E484G G504D V503F Y453F T500N E484A Q493E K417E V503I G485V Q506K P499L K417T G485D R403K G504N G485S G446S G502K N501S K458R V445F V445A P499H G485F S494L Y449H G446V F456L N439D Y495H F490L F486L N501T T478R G496S G446A T478K G485R S494P Q498R Q493R K417N N501I A475S N439K S477G A475V F490S S477R G502N S477I Y489L F490V L455F V445I E484D G446R G446D N501K (in G, GK & GV clade (B.l, B.1.617.2, AY.* & B.1.177); any one or more mutations selected from K417N E484Q Y453F S477G S494L G446V F490L S477I K417T G496S N501Y L455F E484K P499R T478K K458N S494P (in nonG clade (A, B & B.2).

The inventive detection method comprises the step of determining the sequences of the amplified nucleic acids, which stem from the analytes nucleic acids that are processed in the method by amplification steps and wherein the nucleic acid of interest is selected by the primers. The determination of the nucleic acids automatically leads to the identification of the sequence that is amplified. Accordingly, the inventive method can be used to identify known and yet unknown or new variants or strains of a pathogen, such as of SARS-CoV-2. Accordingly, the inventive method in all aspects of the invention may comprise the step of identifying a variant or strain of a pathogen in the samples or analyte nucleic acids. Preferably the variant or strain is of SARS-CoV-2.

In case of SARS-CoV-2 it is particularly preferred when nucleic acid sequences corresponding to amino acids 1-722 and 767-839 of the SARS-CoV-2 spike protein are detected, since in this region many mutations occur that are relevant to the virus' infectivity. In also preferred embodiments, the nucleic acid of interest comprises any of the above-mentioned mutations of variants or strains of SARS-CoV-2 or their location in the SARS-CoV-2 genome, which - as said in the preceding paragraph - allows to identify new variants or strains with new mutations at these genetic positions with the inventive detection method.

Bacterial pathogens may be selected from Neisseria meningitidis, Mycoplasma pneumoniae, Bordetella pertussis, Streptococcus spp., e.g. Streptococcus pneumoniae, a group A streptococcus, Staphylococcus aureus, e.g. Methicillin-resistant Staphylococcus aureus, Mycobacterium tuberculosis, among others.

A fungal pathogen is e.g. selected from Aspergillus spp. (in particular spores thereof).

The pathogen may be a respiratory pathogen, which e.g. can be found in sputum, saliva, nasal mucus, bronchoalveolar lavage. Such body fluids could be collected as samples and pathogens therein could be detected.

Furthermore, the inventive method could be used to detect DNA with a biomarker of a disease as nucleic acid of interest, such as a KrasG12D mutation in DNA shed from lung cancer nodules.

The inventive method can be used to detect any such pathogen or biomarker. The inventive set may comprise primers with a binding sequence for hybridization to the analyte nucleic acids comprising such pathogen nucleic acids or nucleic acids with the biomarker.

In preferments, the sample identifier sequences of the forward primers comprise a sequence distance, preferably a Hamming distance or a Levenshtein distance, to other sample identifier sequences of forward primers of at least 1, preferably of at least 2 or at least 3.

In further preferments, the sample identifier sequences of the reverse primers comprise a sequence distance, preferably a Hamming distance or a Levenshtein distance, to other sample identifier sequences of reverse primers of at least 1, preferably of at least 2 or at least 3.

In preferments, the subset identifier sequences of the further forward primers comprise a sequence distance, preferably a Hamming distance or a Levenshtein distance, to other subset identifier sequences of further forward primers of at least 1, preferably of at least 2 or at least 3.

In further preferments, the subset identifier sequences of the further reverse primers comprise a sequence distance, preferably a Hamming distance or a Levenshtein distance, to other subset identifier sequences of further reverse primers of at least 1, preferably of at least 2 or at least 3.

As mentioned above, the identifier's sequences can be independently selected from the same sequence pools. The nature of being an identifier sequence in the amplified products of step d) being related to the forward, reverse, further forward, or further reverse primer is apparent on the position of the identifier sequence within the amplified products. Amplified products will usually have the structure: [subset identifier from the further forward primer] - [sample identifier sequence from the forward primer] - [analyte sequence] - [sample identifier sequence from the reverse primer] - [subset identifier sequence from the further reverse primer], or any complementary sequence to any part or the entirety of this structure. In dependence of alternate adaptor use, the structure may have the inner part from analyte to sample identifier sequence reversed, such as: [subset identifier from the further forward primer] - [sample identifier sequence from the reverse primer] - [analyte sequence] - [sample identifier sequence from the forward primer] - [subset identifier sequence from the further reverse primer], or any complementary sequence to any part or the entirety of this structure. As such, the position of the identifier sequences can be known and thus using the same pool of sequences for selecting any one of the identifier sequences is non-problematic. Of course, within a group of identifier sequences, no double sequences should occur that would label two different samples identically, or similarly, such as by requiring a sequence distance as mentioned above within a group of identifier sequences. A sequence distance within a group of identifier sequences reduces misassigning in step f), e.g. due to sequencing errors.

In further preferred embodiments, selected independently any or all of the identifier sequences have a length of at least 4, preferably 5, more preferred at least 6 nucleotides, or at least 7 or at least 8, nucleotides. In particular, the sample identifier sequences of the forward primers may have a length of at least 4, preferably at least 5, even more preferred at least 6 nucleotides (nt), preferably at least 7 nucleotides or at least 8 nucleotides, such as 4-16 nucleotides in length; the sample identifier sequences of the reverse primers may have a length of at least 4, preferably at least 5, even more preferred at least 6 nucleotides, preferably at least 7 nucleotides or at least 8 nucleotides, such as 4-16 nucleotides in length; the subset identifier sequences of the further forward primers may have a length of at least 4, preferably at least 5, even more preferred at least 6 nucleotides, preferably at least 7 nucleotides or at least 8 nucleotides, such as 4-16 nucleotides in length; the subset identifier sequences of the further reverse primers may have a length of at least 4, preferably at least 5, even more preferred at least 6 nucleotides, preferably at least 7 nucleotides or at least 8 nucleotides, such as 4-16 nucleotides in length. The length can be scaled according to the numbers of sample processed in parallel in the inventive method in order to maintain distinguishability between the samples and subsets.

The present invention further comprises a set of primers suitable for a method of the invention. The set may comprise or consist of at least 10 different primers A which comprise the sequence, from 5' to 3': an adaptor A sequence, an identifier sequence of at least 4, preferably at least 5, even more preferred at least 6, nt in length and a target binding sequence, wherein the identifier sequence is different within the at least 10 different primers A, preferably with a sequence distance of a Hamming distance of at least 1 or a Levenshtein distance of at least 1, preferably of at least 2 or at least 3. Everything disclosed above regarding to forward primers applies to primers A of the set.

The set may further comprise at least 10 different primers B which comprise the sequence, from 5' to 3': an adaptor B sequence, an identifier sequence of at least 4 nt in length, preferably at least 5, even more preferred at least 6, nt in length, and a target binding sequence, wherein the identifier sequence is different within the at least 10 different primers B, preferably with a sequence distance of a Hamming distance of at least 1, preferably of at least 2 or at least 3, or a Levenshtein distance of at least 1, preferably of at least 2 or at least 3. Everything disclosed above regarding reverse primers applies to primers B of the set.

The set may further comprise at least 10 different primers C which comprise the sequence, from 5' to 3': an adaptor C sequence, an identifier sequence of at least 4 nt in length, preferably at least 5, even more preferred at least 6, nt in length, and a binding sequence that binds to the adaptor A sequence, wherein the identifier sequence is different within the at least 10 different primers C, preferably with a sequence distance of a Hamming distance of at least 1, preferably of at least 2 or at least 3, or a Levenshtein distance of at least 1, preferably of at least 2 or at least 3. Everything disclosed above regarding further forward primers applies to primers C of the set.

The set may further comprise at least 10 different primers D which comprise the sequence, from 5' to 3': an adaptor D sequence, an identifier sequence of at least 4 nt in length, preferably at least 5, even more preferred at least 6, nt in length, and a binding sequence that binds to the adaptor B sequence, wherein the identifier sequence is different within the at least 10 different primers D, preferably with a sequence distance of a Hamming distance of at least 1, preferably of at least 2 or at least 3, or a Levenshtein distance of at least 1, preferably of at least 2 or at least 3. Everything disclosed above regarding further reverse primers applies to primers D of the set.

Primers A may be used as forward primers in the inventive method; primers B may be used as reverse primers in the inventive method; primers C may be used as further forward primers in the inventive method; primers D may be used as further reverse primers in the inventive method.

In preferred embodiments, the target binding sequences of the primers A (or of the forward primers) and primers B (or of the reverse primers) comprise a sequence or antisense-sequence, respectively, of a viral gene. Both primers have a target binding sequence that allows binding of primers to a target nucleotide comprising the target sequence or a complementary sequence thereto (depending if the sense or anti-sense strand is bound). The target binding sequence is also referred to as binding sequence for hybridization to the analyte nucleic acids herein. In the inventive methods, the analyte nucleic acids comprise the target binding sequence or a complementary sequence thereto.

The viral gene preferably is of a SARS-CoV-2 N, M, E or S gene or coding sequence thereof, influenza PA, PB1, PB2, PA, HA, NP, NA, M1, M2, NS1 or NEP gene or coding sequence thereof, a parainfluenza HN, F, M, NP, P or L gene or coding sequence thereof, a rhinovirus VP1, VP2, VP3, VP4, 2A, 2B, 2C, 3A, 3B, 3C or 3D gene or coding sequence; a human respiratory syncytial virus A (HRSV-A) GA1, GA2, GA3, GA4, GA5, GA6, GA7, SAA1, NA1, or NA2 gene or coding sequence; a human respiratory syncytial virus B (HRSV-B) GB1, GA2, GA3, GB4, SAB1, SAB2, SAB3, BA1, BA2, BA3, BA4, BA5, or BA6 gene or coding sequence. These genes or coding regions are also preferred nucleic acids of interest and can be hybridized in step b) of the inventive method with the forward and reverse primers. In particular preferred embodiments of all aspects of the invention, the nucleic acids of interest or viral genes targeted by the primers, are the N gene of SARS-CoV-2, preferably bound or targeted by two different primer pairs, and the influenza M1 or M2 genes. In especially preferred embodiments of all aspects of the invention, the nucleic acids of interest or viral genes targeted by the primers, are the S gene of SARS-CoV-2, preferably bound or targeted by 1, 2, 3, 4, 5, 6, 7, 8, 9, 10, 11, 12, 13 or more, different primer pairs.

The set may further comprise a primer pair that binds to a ribosomal gene or coding sequence or any other internal control of a nucleic acid that is expected to exist in a sample. Such a ribosomal gene or coding region or other internal control may be amplified as a control in parallel to the analyte nucleic acids and its amplification products in step b) and d). The control reaction may be contained in a separate container without analyte nucleic acids or in a container with analyte nucleic acids.

The inventive set may be provided in a kit. Such a kit may be provided in a packaging container comprising the contents of the kit. The kit may also comprise instruction for performing the inventive method.

Furthermore, the kit may comprise any components needed in the inventive method, such as a polymerase, optionally a reverse transcriptase, buffers, nucleotides, a single strand specific nuclease such as a single strand specific RNA nuclease and/or a single strand specific DNA nuclease.

Throughout the present disclosure, the articles "a," "an," and "the" are used herein to refer to one or to more than one (i.e., to at least one) of the grammatical object of the article.

As used herein, words of approximation such as, without limitation, "about", "substantial" or "substantially" refer to a condition that when so modified is understood to not necessarily be absolute or perfect but would be considered close enough to those of ordinary skill in the art to warrant designating the condition as being present. The extent to which the description may vary will depend on how great a change can be instituted and still have one of ordinary skill in the art recognize the modified feature as still having the required characteristics and capabilities of the unmodified feature. In general, but subject to the preceding discussion, a numerical value herein that is modified by a word of approximation such as "about" may vary from the stated value by e.g. ±10%.

As used herein, the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps. The "comprising" expressions when used on an element in combination with a numerical range of a certain value of that element means that the element is limited to that range and "comprising" relates to the optional presence of other elements. E.g. the element with a range may be subject to an implicit proviso excluding the presence of that element in an amount outside of that range. As used herein, the phrase "consisting essentially of" requires the specified integer(s) or steps as well as those that do not materially affect the character or function of the claimed invention. As used herein, the closed term "consisting" is used to indicate the presence of the recited elements only.

The present invention will be further described by the figures and examples, without being necessarily limited to these embodiments of the invention.

### Figures

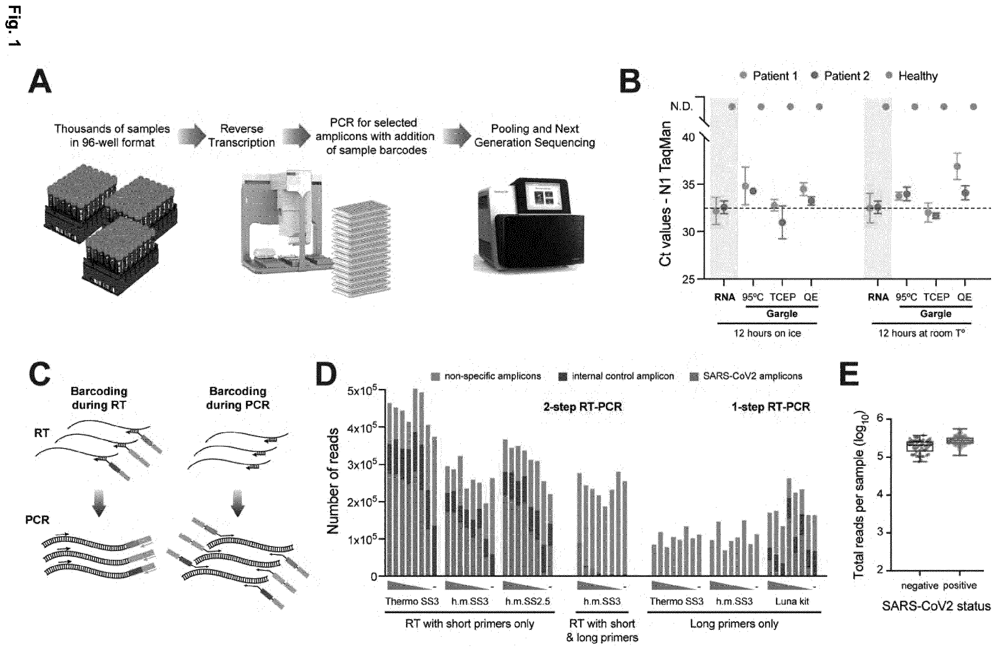

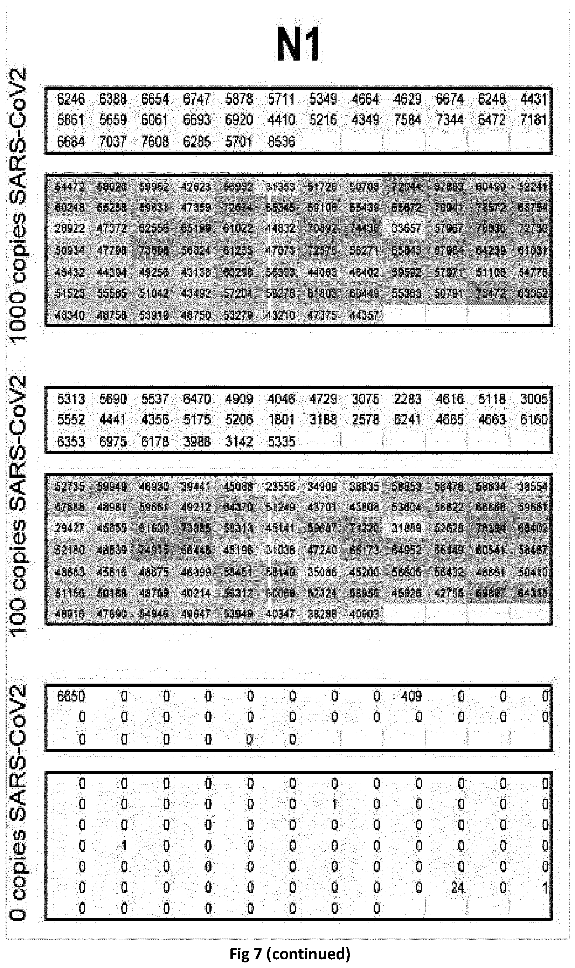

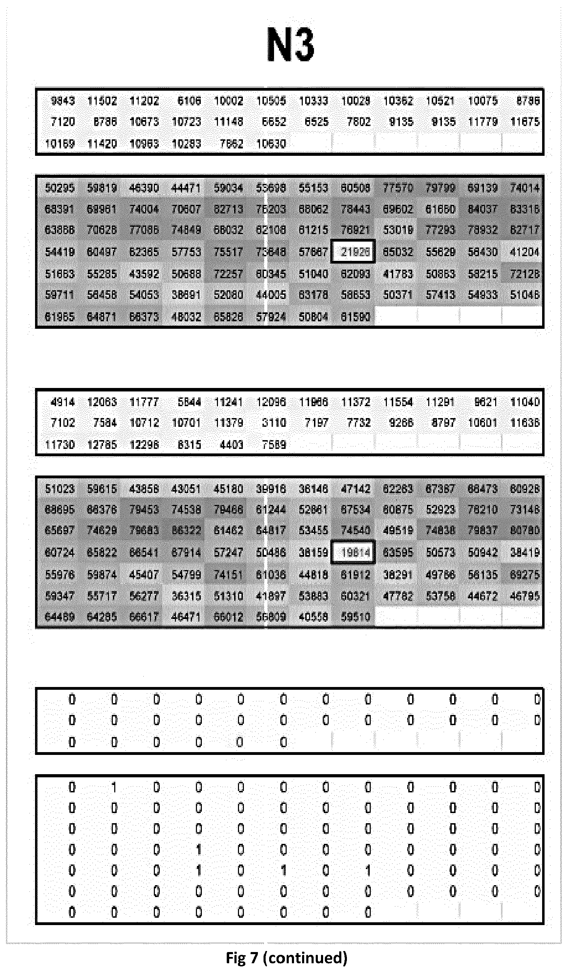

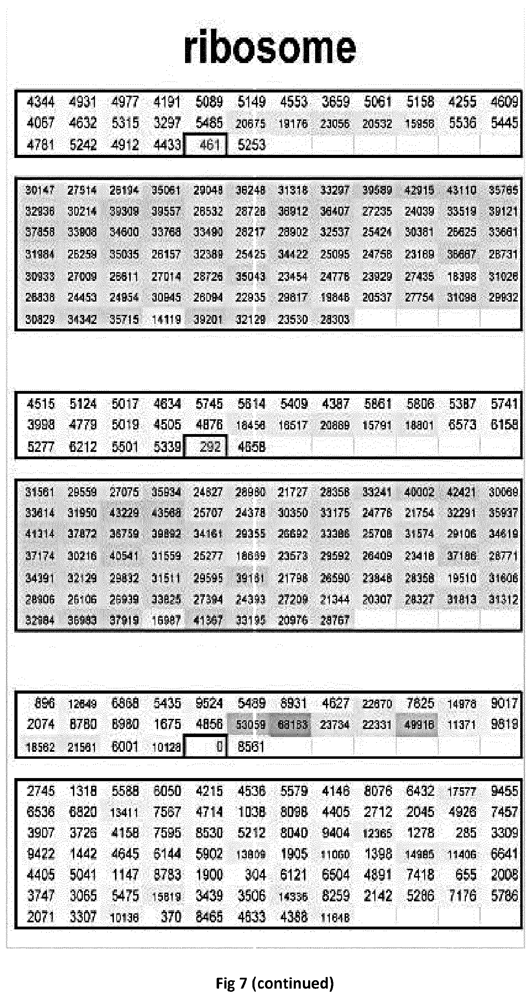

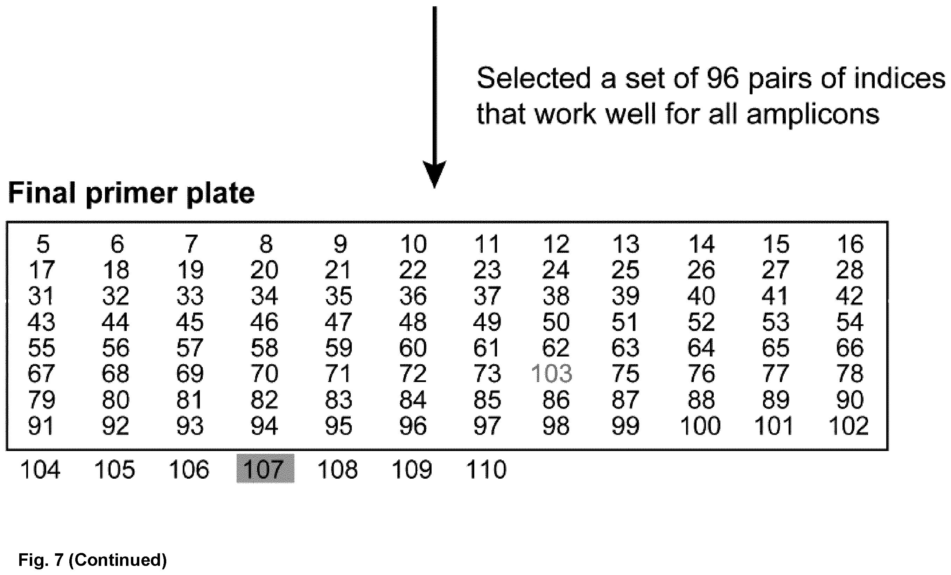

**Figure 1****: Setup of a next generation sequencing (NGS) based pipeline for the detection of Sars-CoV2 from crude respiratory samples. A**. Overview scheme to illustrate the envisioned analysis pipeline. **B**. Comparative analysis of SARS-CoV2 detection following different RNA extraction treatments by TaqMan qRT-PCR on the N1 amplicon. Gargle samples from two SARS-CoV2 positive and one negative person were collected in HBSS. RNA was purified (without concentrating) or crude lysates were produced using either heat inactivation for 30 min (REF), TCEP/EDTA (HUDSON buffer), or QuickExtract. **C**. Illustration of amplicon indexing either during RT or PCR. Colored boxes symbolize different indexes to be used in individual samples for detection during NGS. Arrows illustrate oligonucleotides hybridizing to RNA during RT or DNA in PCR. **D.** Comparison of amplicons obtained upon indexing in RT or PCR. Long primers containing indexes and complementary sequences of >20 bp and thus compatible with PCR were either included as RT primers already, or RT was performed with short primers, i.e., random hexamers and two N gene-specific primers binding the 3' of the SARS-CoV2 amplicon. In 1-step PCR, hot start Taq polymerase was already present during RT, while in 2-step PCR hot start Taq polymerase was added subsequent to RT. Grey triangle symbolizes a dilution series of synthetic template of 3645, 1215, 405, 135, 45, 15, 5, or 0 molecules. Thermo SS3: Superscript III enzyme provided by Thermofisher; h.m. SS3: homemade superscript III like enzyme; h.m. SS2.5: homemade advanced superscript II like enzyme; E. Comparison of total read counts obtained from a total of 192 samples either negative or positive for SARS-CoV2 at various concentrations.

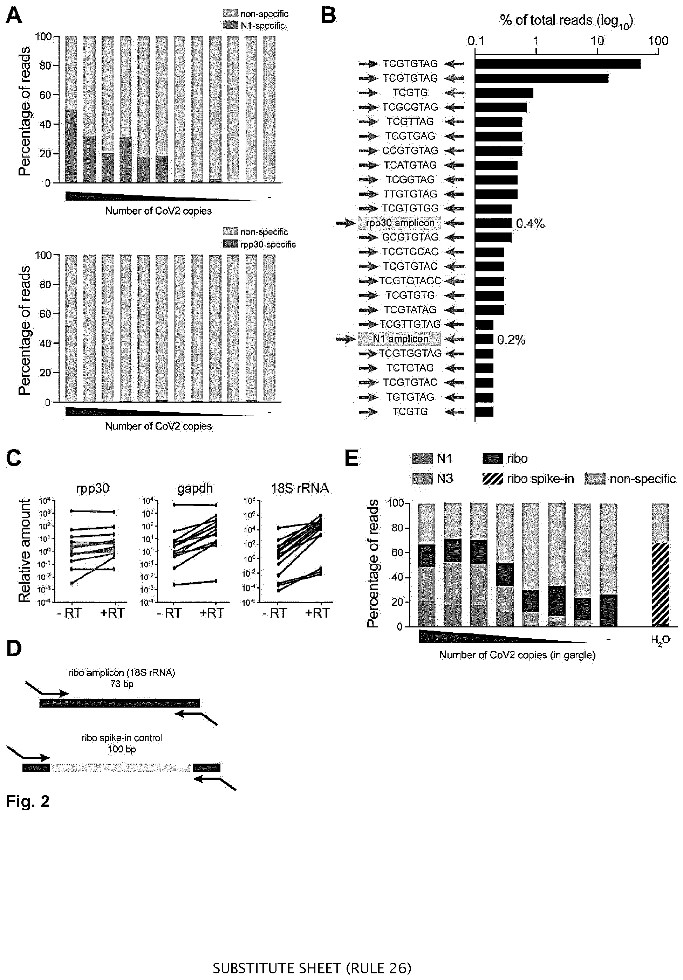

**Figure 2****: A control primer pair targeting 18S rRNA provides better specificity than the widely used RPP30 primers.** A. NGS read counts obtained with the SARS-CoV2 N gene-specific primer pair N1 as well as the human RNAse P (RPP30)-specific primer pair designed by the CDC. RT-PCR was performed on inactivated gargle-QE samples spiked with 5120, 2560, 1280, ..., 5 molecules of synthetic template in a twofold dilution series as well as 0 molecules. Non-specific amplicons are defined as amplicons generated by the respective matched primer pairs but with incorrect sequence in between. **B.** Analysis of all amplicons generated in the pool of conditions shown in A. Unspecific amplicons typically incorporated a short stretch of sequence complementary to primer sequence and were almost exclusively generated by at least one RPP30-specific primer. Specific amplicons add up to <1% of reads in this condition. **C.** SYBR Green qPCR analysis of RPP30 primers and two alternative internal control primer pairs in the presence and absence of RT on RNA purified from gargle of 16 individuals. Ct values are transformed to arbitrary units. **D.,E**. Illustration of an RT control (RTC) spike-in; An amplicon with identical primer binding sites to the ribosomal internal control yet different and longer intermediate sequence was synthesized, cloned, and T7 transcribed. It was added to the reverse transcription at 1000 molecules/reaction. The ratio of ribosomal versus RTC reads serves as a sample quality measurement, reads for neither the ribosome nor the spike-in point towards an inhibited/failed PCR reaction for the specific sample.

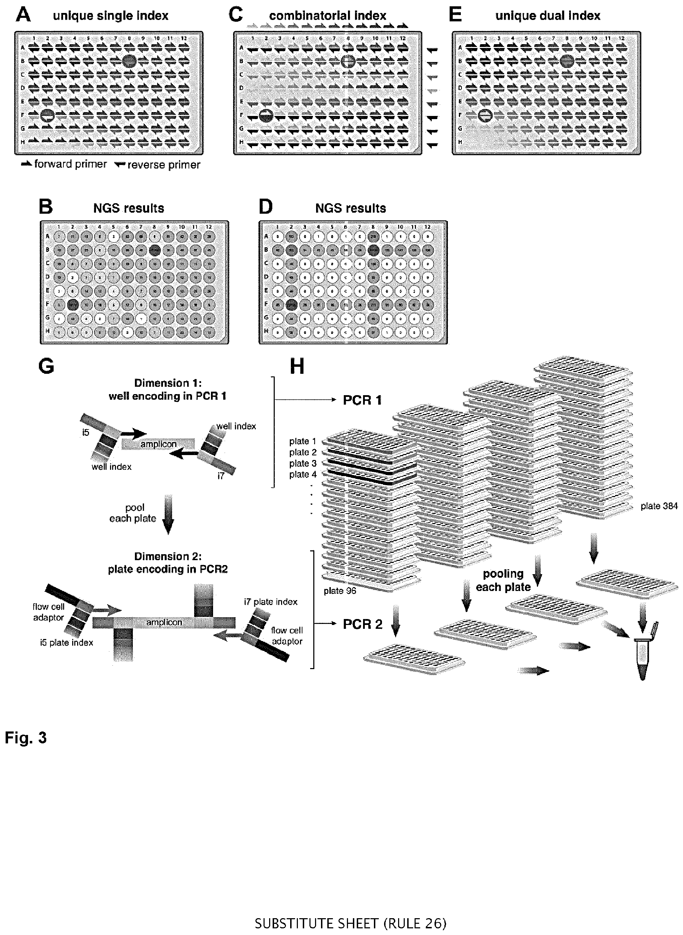

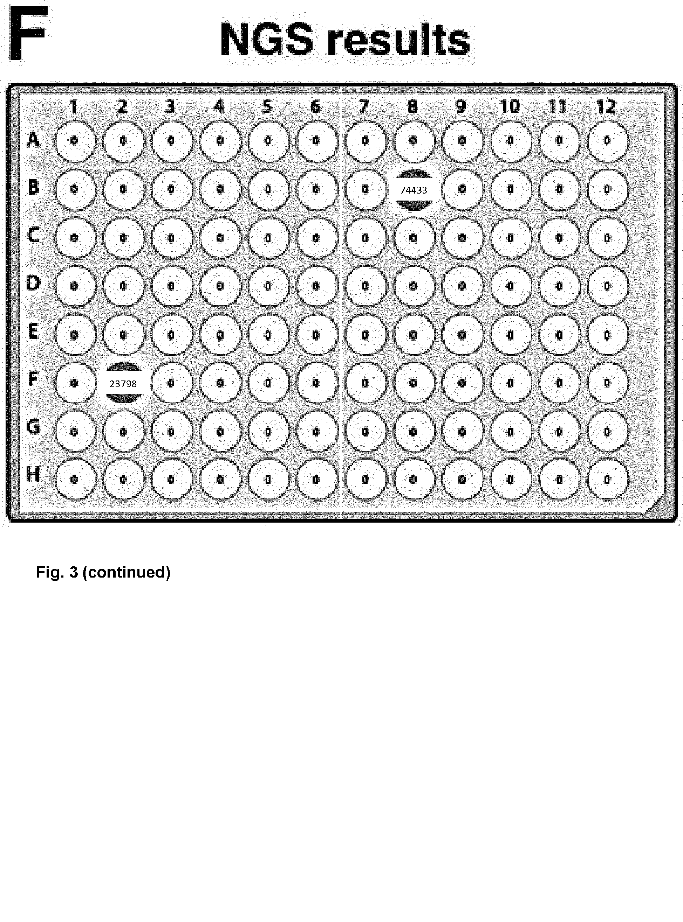

**Figure 3****: Two-dimensional redundant dual indexing allows scaling to population-level testing. A.** Scheme depicting 96 well-based indices. These can be incorporated by forward primers or reverse primers. For the latter they can be incorporated during RT or PCR. Red circles highlight the positions into which synthetic template was loaded to test performance of the indexing strategy. **B.** N3 amplicon reads obtained from next generation sequencing mapped to wells based on indices incorporated by reverse primers during PCR as in A. Forward primer indices were disregarded in this analysis. Note the frequent mis-assignment to incorrect positions. **C.** Scheme depicting combinatorial indexing. Each well is identified as a unique combination of forward and reverse index. **D.** N3 NGS reads mapped to wells based on combinatorial indexing as in C. To simulate combinatorial indexing the identical dataset as in B and F was used and primers were treated in pools pointing to columns or rows. **E.** Unique dual indexing is a redundant indexing method that encodes e.g. wells both by forward and reverse primers. Thus, illegitimate recombination products of reads can be bioinformatically rejected. **F.** NGS result for the same dataset as in B and D analyzed with dual indices as in E successfully filtered away all misassigned reads. **G.** Two-step PCR strategy to integrate redundant dual indices pointing to wells and subsequently plates. Colored boxes represent indices. **H.** Illustration of the PCR workflow. RT and PCR 1 are performed on all samples individually. Subsequently each plate is pooled to one well position, the amplicon is treated with Exostar to remove excess primers, and a second PCR is done. Our currently used and validated barcode set allows pooling of up to 36,864 samples. All PCR 2 amplicons are pooled, gel purified, and sequenced on any Illumina platform.

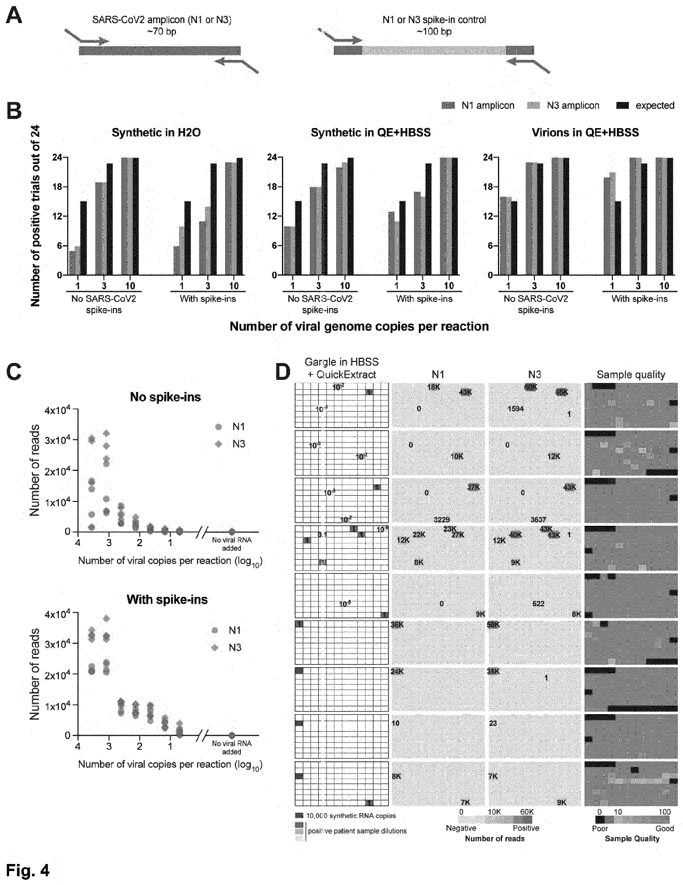

**Figure 4****: SARSeq is specific and sensitive when tested on a large set of gargle samples. A.** Schematic illustration of RNA spike-ins to scavenge N1 and N3 primers during PCR 1 in SARS-CoV2 negative samples. Analogous to the RTC, primer binding sites are identical to N1/3 amplicons, but intermediate sequence is distinct and longer so as not to compete with virus-derived amplicons. **B.** Frequency of detection of SARS-CoV2 at minimal template concentration. Number of detected cases of 24 is depicted. Note that the expected frequency of detection is only 63% and 95% for one and three molecules assuming a Poisson distribution of molecules in wells. **C.** Read counts of quadruplicates in dependency of synthetic SARS-CoV2 template. Note the reduced variability in the presence of N1/3 spike-ins. **D.** SARSeq performance on a test pool of 864 samples. Gargle samples collected in HBSS for our in-house testing pipeline of personnel were spiked with synthetic RNA or a dilution series generated from a positive sample presenting undiluted with a Ct of 30. All missed samples were 1:100,000 dilutions of that probe and thus presumably negative for SARS-CoV2. No negative positions presented with >1 read. Sample quality is assessed by the ratio or ribosomal reads to ribosomal spike-in.

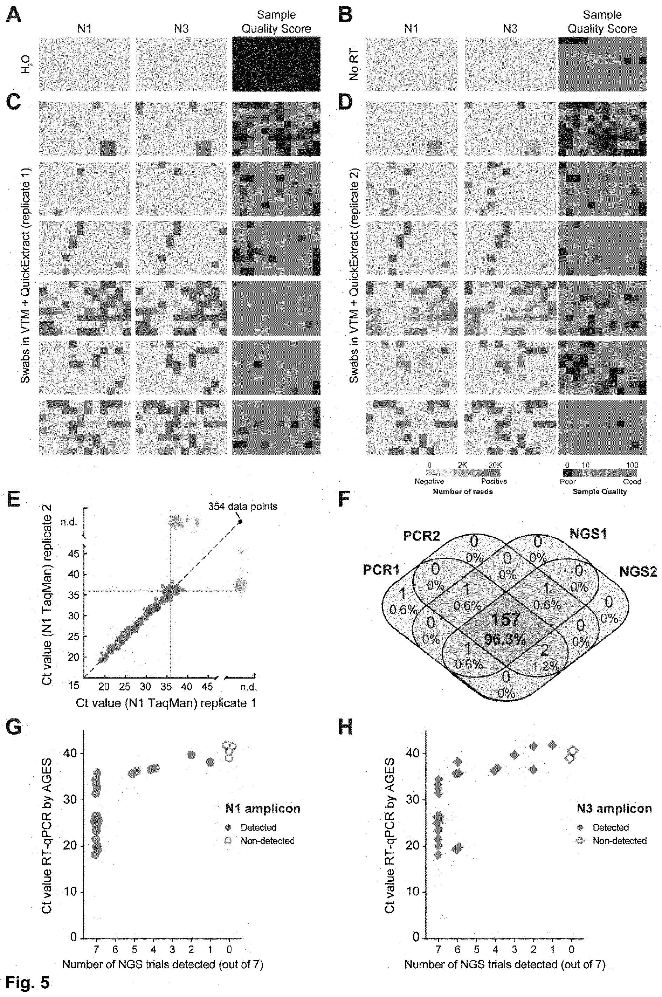

**Figure 5****: Validation of SARSeq on patient samples. A.** H₂O control plate of SARSeq with three primer pairs, namely N1, N3, and ribosome in the presence of RTC as well as N1 and N3 scavenger spike-ins. **B.** SARSeq on a gargle-QuickExtract plate in the absence of reverse transcriptase. No false positive wells were detected. Sample QC scores high due to the absence of RTC reads while DNA templated ribosomal amplicon is observed. **C.** Analysis of 576 samples obtained by swab and collected in VTM. **D.** Independent replicate of C based on the same inactivated patient swab. Color codes depict read counts and sample quality score for C and D. **E.** Two independent N1 TaqMan qPCR runs on samples as in C and D. Ct values of both runs are plotted against each other. Color code: red: sample scoring positive in both qPCR replicates, orange: sample scoring once, black: sample scoring negative. Stochastic and latest detection of SARS-CoV2 at cycle 36. **F.** Venn diagram of reproducibility for all samples scoring positive with Ct 36 or less for at least one qPCR replicate. **G, H.** Comparison of NGS results by SARSeq to Ct values obtained by diagnostic qPCR. A set of 90 samples (including swabs and gargle in different buffers) was used for RNA extraction and qPCR measurements and in parallel aliquots of these samples were mixed either with QuickExtract or TCEP/EDTA and measured by SARSeq in triplicates and quadruplicates, respectively. We report the number of replicates in which we called a sample positive by NGS (with the N1 or N3 amplicon) relative to the qPCR Ct values. Not shown are 63 samples that were negative by qPCR and NGS.

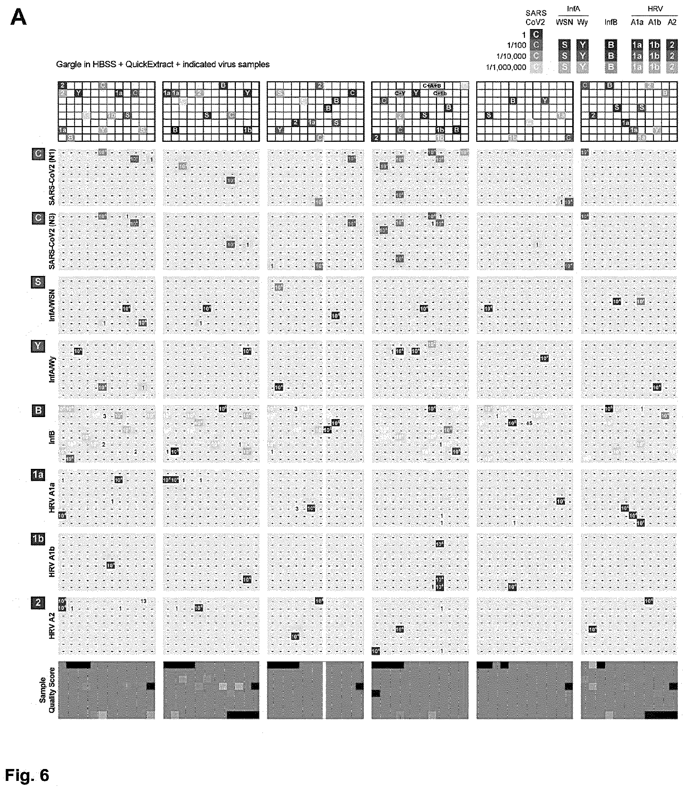

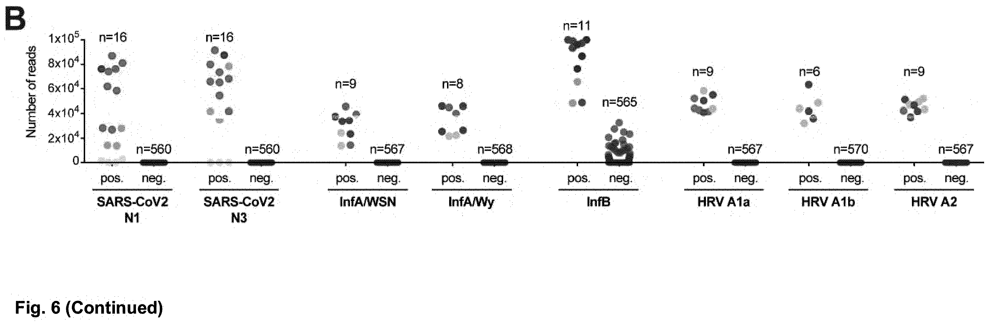

**Figure 6****: Multiplexed analysis of several respiratory viruses. A.** Six 96 well plates filled with gargle in HBSS and inactivated in QuickExtract from our in-house pipeline were spiked with RNA from various respiratory viruses. For SARS-CoV2, a positive gargle sample with Ct value of 30 was diluted as indicated. RNA for all other viruses was obtained from HEK cells 48 h after infection with the virus. Dilution indicated by volumetric ratio. SARSeq was performed with six primer pairs in one reaction, namely N1 and N3 for SARS-CoV2, Influenza A virus, Influenza B virus, human rhinovirus (HRV), and the ribosome. Influenza A substrains and HRV substrains were distinguished based on amplicon sequence variants. **B.** Analysis of false positive and false negative rate of the experiment in panel A. As expected, 1:100,000 dilutions of the SARS-CoV2 sample with a Ct of 30 were missed. All other positive samples were detected for all viral spike-ins. False positive samples were detected for Influenza B virus presumably due to sporadic contamination of reagents or equipment with purified RNA also in the H₂0 control plate (not shown). False positive read counts did not reach numbers observed in truly positive samples. No false positive samples above threshold were detected for any other virus.

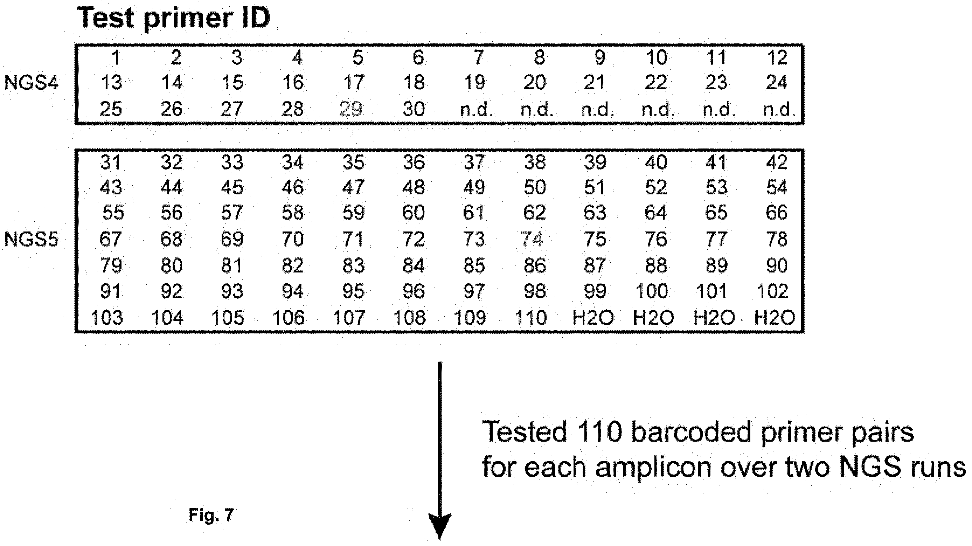

**Figure 7****:** Testing of primer pairs for three amplicons (SARS-CoV2 N1 and N3, and human ribosome) carrying extensions with 110 unique dual indexes. Primer pairs with indices #29 were excluded as they did not efficiently produce the ribosome amplicon; primer pairs with indices #74 were excluded as they did not efficiently produce the N3 amplicon. Primer pairs 1-4 can be used efficiently but were not included in the final set because these indices were frequently used in the lab during initial setup of the method and we wanted to eliminate all possible sources of cross contamination.

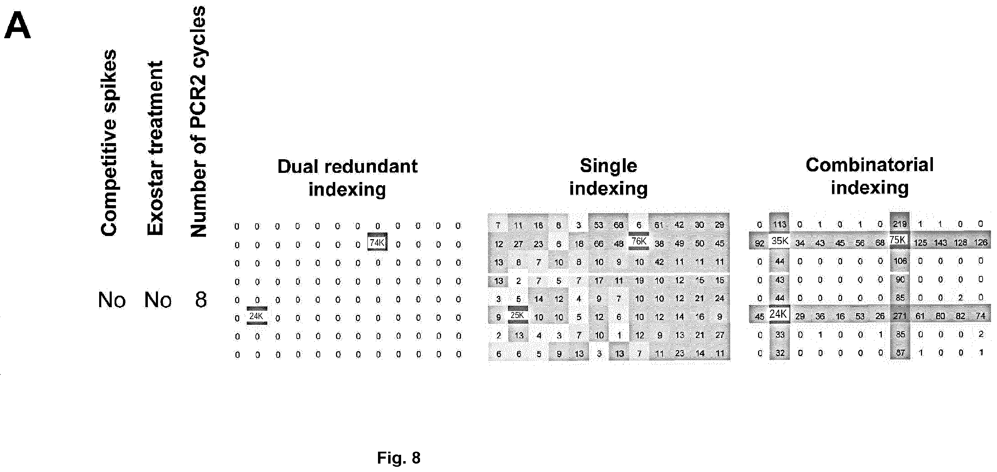

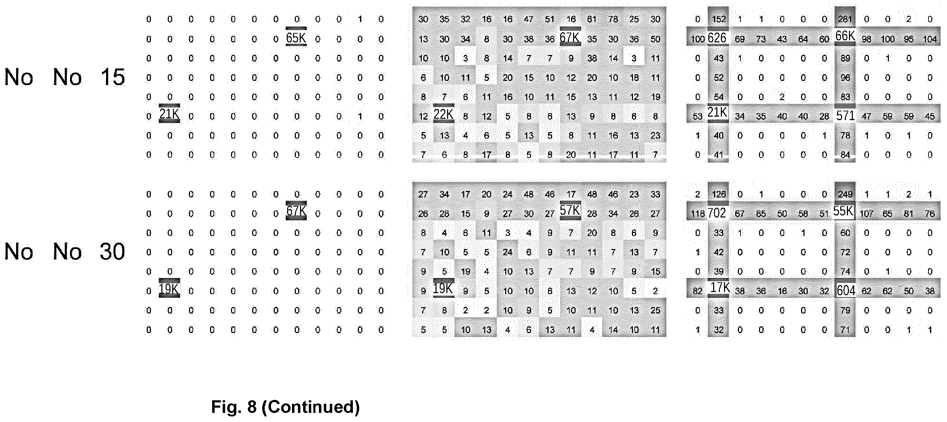

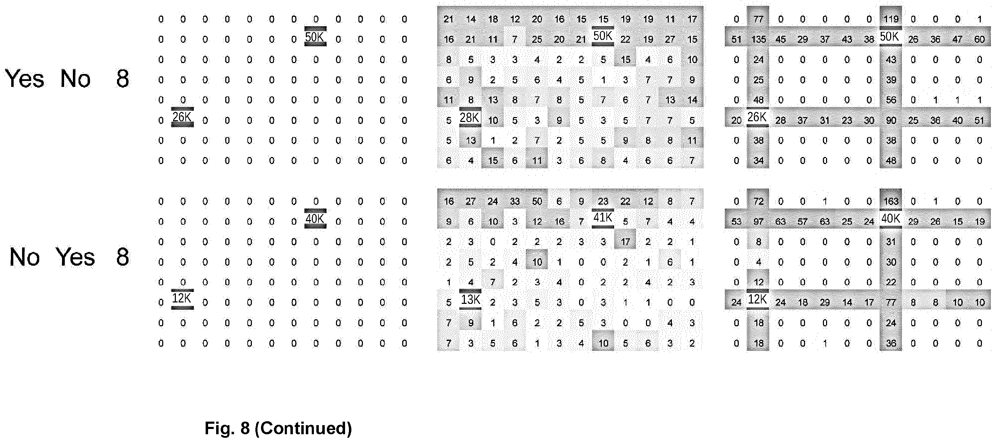

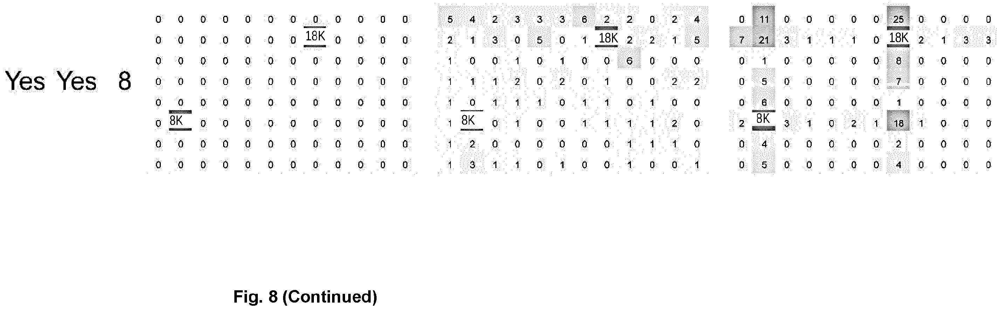

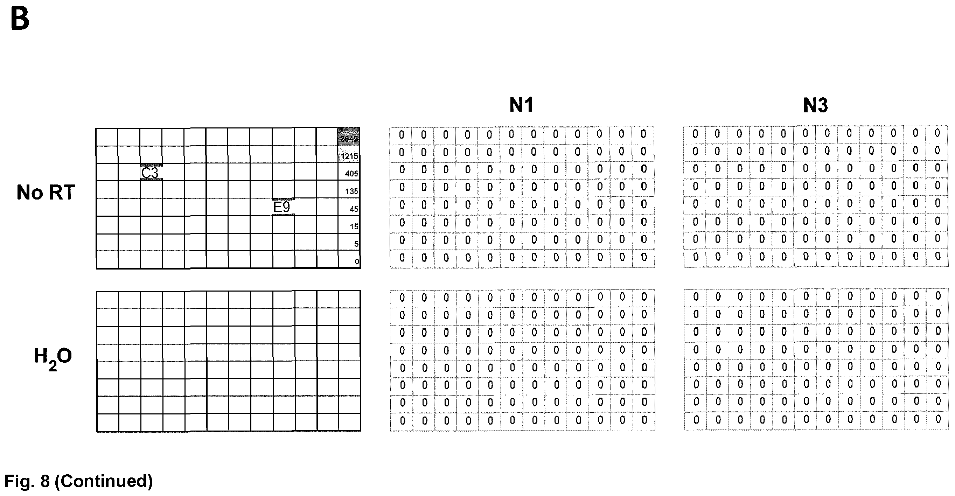

**Figure 8****: A.** Contribution of the different measures taken to completely suppress mis-assignment of indices to incorrect samples (and in that way avoid false positives). SARS-CoV2 synthetic RNA was added to positions B8 and F2. Assigning sample identities based on single indices or combinatorial indices produces a substantial amount of misassignments. These can be reduced with three independent measures: addition of a competitive spike-in and treatment with Exostar to remove any left-over primers after PCR1, and limiting the number of cycles in PCR2 to prevent recombination between amplicons and their indices. Whereas all these measures help reduce misassignments, adding dual redundant indices (or unique dual indices) completely suppresses this. **B.** Shown are N1 and N3 reads for two negative control plates (-RT and H₂O) that were run together with numerous other positive plates in the same run. The fact that we detect 0 reads shows that there is no misassignment of well identities across plates either. This is because each plate is also encoded by redundant/unique dual indices.

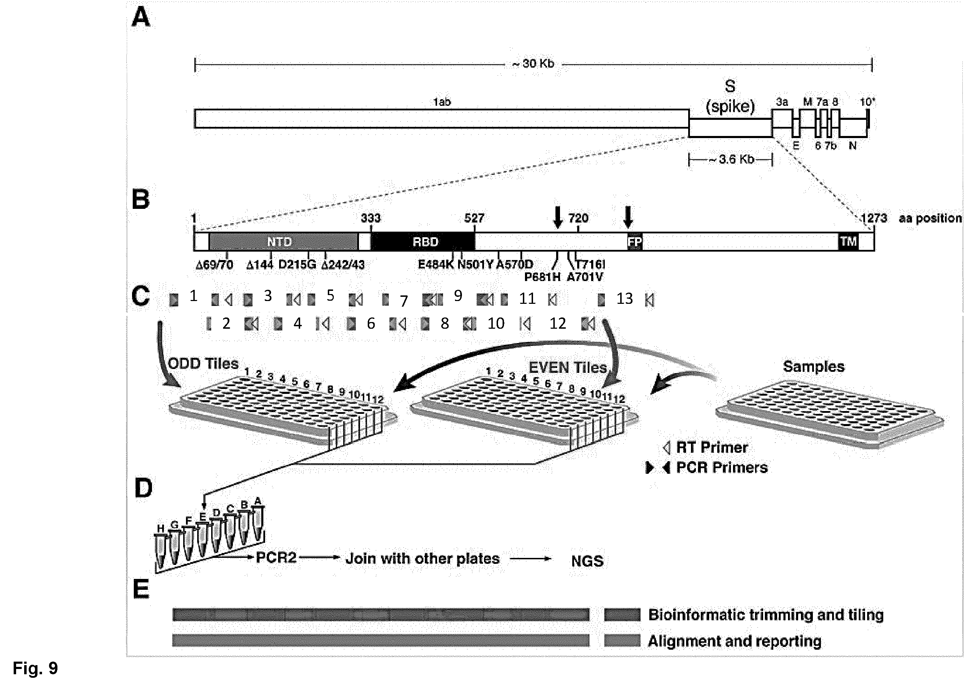

**Figure 9****:** SARSeq spike protein variant detection. Viral genomic RNA is reverse transcribed with 13 primer pairs that are specific to the S gene and the resulting cDNA is amplified in 13 partially overlapping fragments or "tiles" that together cover about two thirds of the S-gene (**Fig. A and B**). Specifically, the part of the S-gene encoding the extracellular part of the Spike protein is covered, where interaction with the ACE2 receptor and the majority of the known neutralizing antibodies occurs. This is also the part where all current mutations of concern have been identified (**Fig. B**). The critical region from amino acids (aa) 470-502 is covered twice (by tiles 8 and 9) to ensure robust detection of variants of particular interest. Because overlapping fragments cannot be amplified together in the same reaction tube (as primers would then preferentially amplify the overlap), the RT and PCR are practically conducted in two separate reactions (**Fig. C**) which are referred to as "odd" and "even" referring to the numbers of the tiles included. After RT and PCR1, samples are pooled, subjected to PCR2 to add the second set of barcodes and eventually pooled with samples from other plates to be sequenced (**Fig. C and D**). The tiles are bioinformatically aligned to extract a consensus sequence that can be compared to the reference S-gene sequence to identify variants.

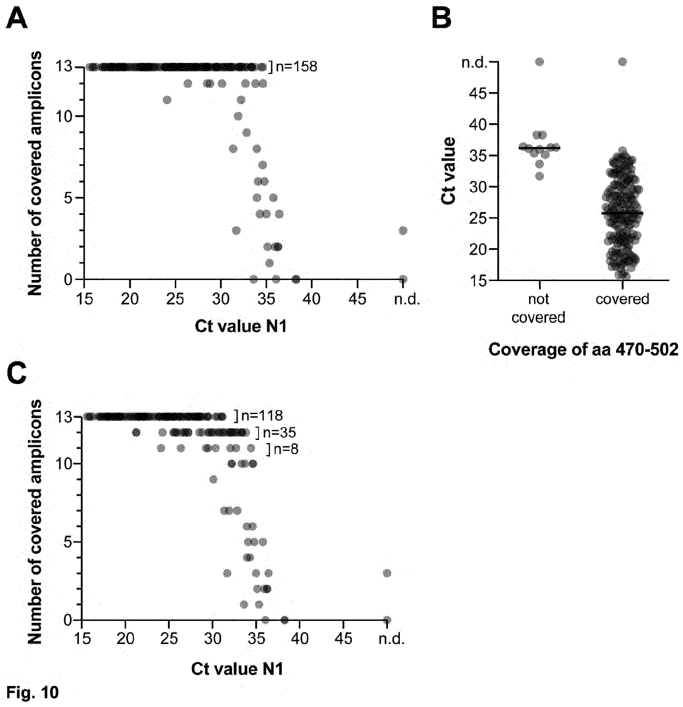

**Figure 10****: SARSeq sequence recovery efficiency;** For 192 positive samples qPCR Ct values using a commercial one-step kit (LUNA) were compared to amplicon coverage by SARSeq. Cutoff for recovery is 2% of median number of reads for that amplicon or a minimum of 10 reads. Sequencing data are obtained as a pool of 2255 on one NovaSeq lane. (A) Recovery of up to 13 amplicons per sample in dependence of Ct value. Sequence recovery is almost complete until a Ct of 33. (B) Sequence coverage at the most variable domain (amino acid positions 470-502) of the S-gene by at least amplicon 8 or 9. Almost all samples <Ct35 can be analyzed for the relevant mutations S477N, E484K, N501Y and other mutations in the region. (C) Due to the efficient and specific sequence retrieval and the focus on the S-gene as well as a robust barcoding strategy, SARSeq enables multiplexing of >20k samples into a single NovaSeq lane for sequence analysis. The plot estimates sequencing efficiency of >20000 samples together by setting the cutoff 10x higher, namely to 100 reads.

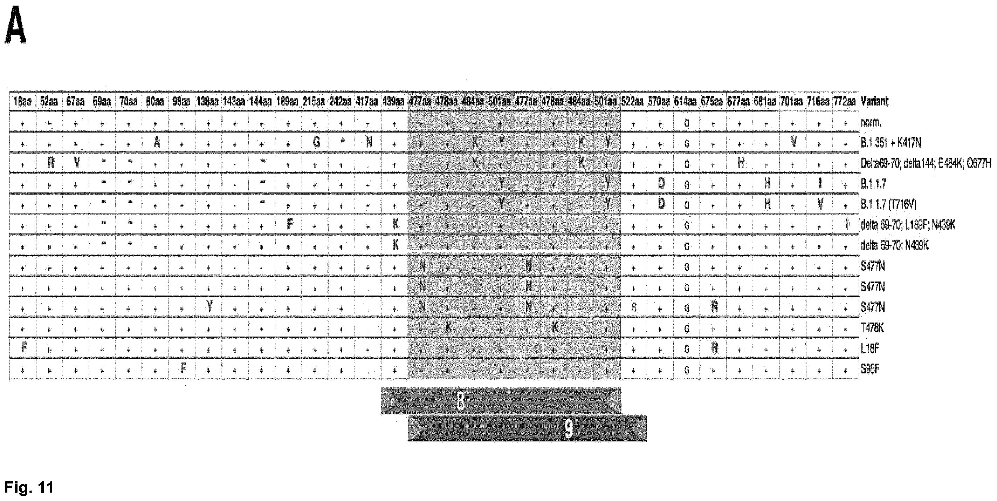

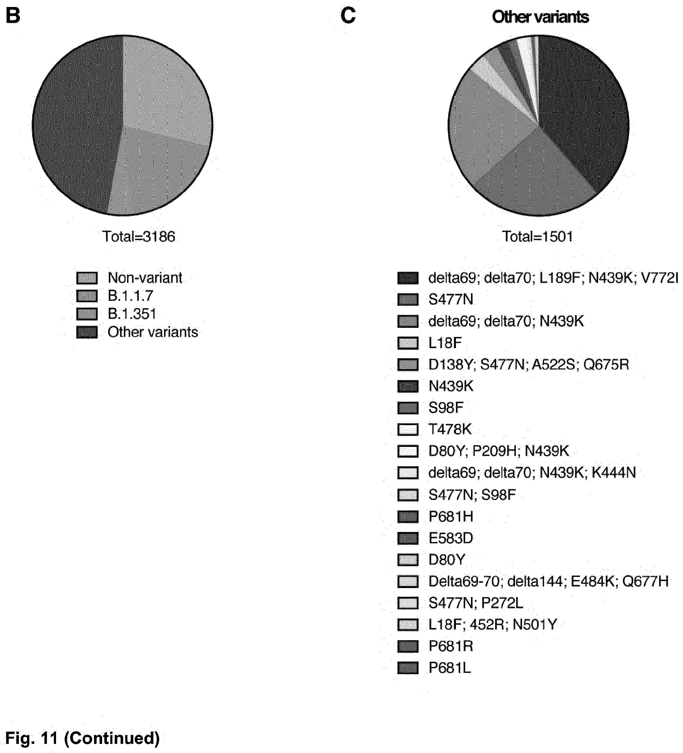

**Figure 11****: SARSeq output exemplified on one analysis batch;** (A) Example of a tabular output of the analysis pipeline highlighting a few relevant amino acids. The total analysis window covers amino acids 1-722 and 767-839, see primers in example 23. (B) Pie chart of variant distribution highlighting the two variants of concern B.1.1.7 and B.1.351 versus strains with minor or other mutations. (C) Breakdown of other variants with annotation of observed amino acid changes.

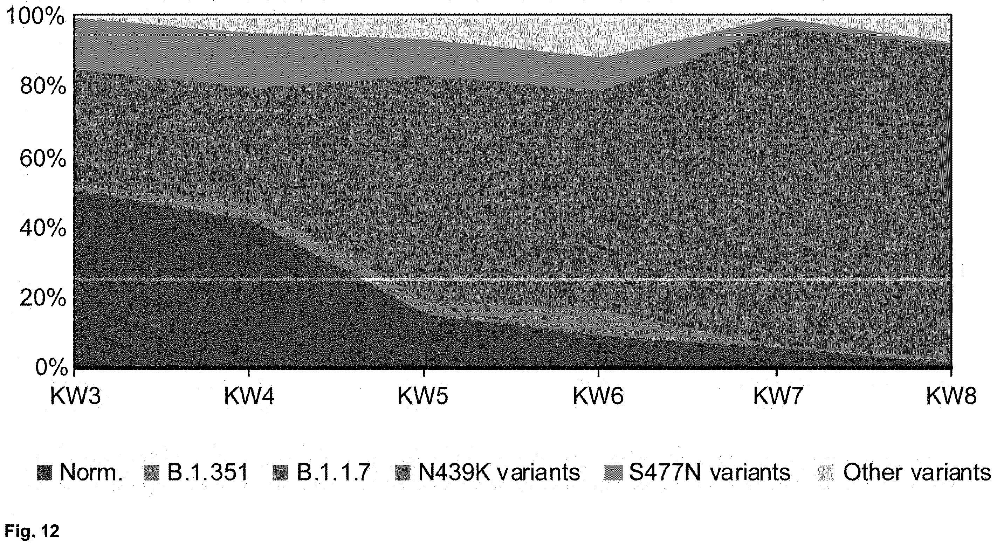

**Figure 12****: Timeline of variant distribution observed in SARSeq analysis runs**; Mutation frequency within analyzed samples per week (calendar week of 2021, KW) is plotted. Each time point consists of 2200-2500 analyzed samples.

### Examples

The invention provides a sensitive and specific method to detect SARS-CoV2 RNAs and other nucleic acids that could be scaled to tens of thousands of parallel tests. The gold standard to detect SARS-CoV2 are RT-qPCR-based assays, which measure the abundance of short RNA fragments characteristic of SARS-CoV2 by means of DNA amplification. As PCR reactions can amplify incorrect fragments (unspecific amplicons), despite the use of specific primer pairs, RT-qPCR assays typically increase specificity by the use of fluorescently labeled TaqMan probes. This typically means that only a few different amplicons can be detected per reaction, which - together with the need for specific qPCR machines - severely limits the scalability and throughput of these assays (especially if sample-pooling is not desirable).

Next-Generation-Sequencing (NGS) can in principle allow the detection of specific amplicons by sequencing and computational analyses and is not limited in the number of different amplicons it can detect: as the amplicon identity is revealed during sequencing, multiple different fragments (viral and cellular controls) can be amplified in the same reaction if the primer pair used for the different amplicons are compatible. In addition to detecting multiple different amplicons per sample in parallel, individual samples can be uniquely labeled with characteristic sequence-barcodes, allowing the subsequent pooling and pooled sequencing. The advantages of detecting multiple amplicons per sample and processing tens of thousands of samples in parallel mean that NGS-protocols offer huge cost-saving potential and are thus highly attractive for large-scale testing.

However, while NGS-protocols are conceptually simple and indeed several different protocols have been proposed, their efficient implementation is challenged at various steps and these challenges directly impact general feasibility, costs and potential cost savings.

Here, we describe a robust high-throughput protocol based on a redundant, dual and two-dimensional barcoding strategy that achieves perfect sample-recall by redundant dual indexing while scaling to tens-of-thousands of samples by combinatorial indexing along two dimensions, termed SARSeq (Saliva Analysis by RNA Sequencing, according to one of its preferred uses). Example protocols further use a 2-step endpoint RT-PCR and using NGS-compatible amplicons and primer pairs. The protocols were validated on samples with synthetic RNAs and on patient samples, demonstrating that the protocols' utility extends to the simultaneous detection of SARS-CoV2, influenza and HRV viruses from the same sample in a single experiment. Overall, the pipeline can be efficiently combined with high-throughput sample collection in e.g. 96- or 384-well formats, robotics and NGS to detect SARS-CoV2 and other viruses in tens of thousands of samples per experiment with a turn-around time of about 1 day (Figure 1A).

### Example 1: Input sample preparation

The pipeline we describe can start from a variety of different input samples. The types of tested samples:
- Purified RNA from gargle samples
- Gargle samples mixed with QuickExtract solution form Lucigen (1:1 ratio)
- Swabs in VTM mixed with QuickExtract (1:1 ratio)
Samples mixed directly with QuickExtract were incubated for 5 min at 95°C for inactivation and used directly or stored frozen at -80°C. We did not observe a decline in positive signals upon freezing and thawing (even after two cycles of freeze-thaw).

The samples were arrayed in 96-well plates. The described reaction setup uses up to 5 µL of any of the above described samples.

### Example 2: Reverse transcription

Reverse transcription was performed with Superscript III reverse transcriptase and a primer mix containing random hexamers as well as two 12-mer oligonucleotides that prime on the SARS-CoV2 N gene.

A master mix containing all components listed below was prepared and distributed to 96-well plates (20 µL per well). Using a liquid-handling robot (or multi-channel pipettes), 5 µL of each sample were transferred to individual wells containing the RT reaction mix. RT reactions were set up at room temperature. Plates were sealed with aluminum sealing foil (It facilitates easy removal after RT reaction that reduces vibrations in wells avoiding generation of aerosols which may cause cross contamination between samples) and incubated in a thermocycler following conditions listed below.

### Master mix composition per reaction/well (volumes in µL):

| | |
|---|---|
| 10x RT Buffer^{A} | 2.5 |
| 25 mM each dNTPs | 0.5 |
| 1M DTT | 0.1 |
| RT primer mix^{B} | 2 |
| RNAse Inhibitor | 0.5 |
| Superscript III | 0.5 |
| Water | 13.8 |

Wherever mentioned, for each reaction 1000 copies of Ribosome synthetic RNA spike-in and 50 copies of each N1 and N3 RNA synthetic spike-in is included in the RT master mix. Also, wherever mentioned Thermofisher/Invitrogen^{™} SuperScript^{™} III or Luna Universal One-Step RT-qPCR Kit (NEB) or Superscript 2.5 is used for reverse transcription. In all other experiments Superscript III is used for reverse transcription.

### Thermocycler program:

5 min at 25°C (primer annealing)
15 min at 55°C (reverse transcription)
3 min at 95°C (RT inactivation)
Cool down to 12°C (removing the plate while it's still hot will cause bending of the plastic, making further pipetting and sealing more difficult)

### ^{A}10x RT Buffer composition:

200 mM Tris-HCl pH 8.3
500 mM KCl
50 mM MgCl₂
200 mM (NH₄) ₂SO₄
1% Triton X-100
*^{B}RT primer mix composition:* 12.5 mM of each random hexamers, N-gene specific 12-mer #1 and N-gene specific 12-mer #2 (final concentration in the complete 25 µL RT reaction is 1 mM each)

### Example 3: In vitro transcription of spike-in synthetic RNA templates

Ribo, N1 and N3 spike-in DNA fragments with unique identification sequences in the middle and away from primer binding sites are ordered from IDT or Microsynth. Spike-in templates are cloned into pCR2.1 plasmid by topo cloning. Spike-in template containing plasmid clones are confirmed with Sanger sequencing. For efficient in vitro transcription, plasmids are linearized downstream of the T7 promoter and spike-in template by cutting with a unique restriction enzyme. *In vitro* transcription is carried out using NEB HiScribe^{™} kit according to manufacturer's instructions. Transcribed reaction was treated with Turbo DNAse/Thermofisher for 1 hr and RNA is purified using Zymo RNA clean and concentrator spin columns. RNA is aliquoted and stored at -80C.

### Example 4: First PCR

A first PCR was performed for amplification of cDNA with addition of sample indexes as dual barcodes.

A master mix containing all components listed below, including HotStart Taq Polymerase and Uracil DNA glycosylase (Antarctic Thermolabile UDG from NEB/M0372L) was prepared and distributed to a deep-well 96-well plate. The 96 primer pair combinations^{C} containing dual well barcodes were also arrayed in 96-well plates (multiple primer plates can be prepared simultaneously and stored frozen at -20°C). Using a liquid-handling robot, the 96 sets of barcoded primers were added to the PCR master mix and mixed thoroughly. 25 µL of this complete 2x PCR mix were added to the 25 µL RT reactions prepared as above. Plates were sealed with aluminum sealing foil and incubated in a thermocycler following conditions listed below.

All components were kept at room temperature during reaction set up; together with the first step in the thermocycler, a 10 min incubation at 30°C, this provides the right conditions for UDG to act on Uracil-containing amplification products of previous PCR reactions, thereby removing spurious carry over contaminants. After UDG heat inactivation, the subsequent PCR reaction was again carried out in the presence of UTP to prevent carry over contamination in following runs.

### Forward primers:

1^{st} set for the SarsCoV2 N gene (N1): SEQ ID NO: 1 to 96
2^{nd} set for the SarsCoV2 N gene (N3): SEQ ID NO: 97 to 192
Set for rRNA: SEQ ID NO: 193 to 288
Set for influenza A: SEQ ID NO 289 to 384
Set for influenza B: SEQ ID NO: 385 to 480
Set for human rhino virus: SEQ ID NO: 481 to 495

All forward primers contain the same 5' part containing the adaptor sequence (CTACACGACGCTCTTCCGATCT (SEQ ID NO: 991) - nt 1 to 22 of any of SEQ ID NO: 1 to 495) followed be the staggering nucleotides, i.e. 1 to 4 N, which are randomly selected from A, C, G, or T, followed by the sample identifier sequence, which is different for all primers within a given amplicon-specific set, here a 8-mer for all of SEQ ID NO: 1 to 495 (e.g. CCAATACT (SEQ ID NO: 992), nt 24-31 of SEQ ID NO: 1), followed by a binding sequence for hybridization to the analyte nucleic acids; e.g. for N1: gaccccaaaatcagcgaaat (SEQ ID NO: 993) (nt 32 to 51 of SEQ ID NO: 1); for N3: gggagccttgaatacaccaaaa (SEQ ID NO: 994) (nt 32 to 53 of SEQ ID NO: 97); for rRNA: ggcattcgtattgcgccgcta (SEQ ID NO: 995) (nt 32 to 52 of SEQ ID NO: 193); for influenza A: CTCATGGARTGGCTAAAG (SEQ ID NO: 996) (nt 32 to 49 of SEQ ID NO: 289); for influenza B: GCCTTCTCCATCTTCTG (SEQ ID NO: 997) (nt 32 to 48 of SEQ ID NO: 385); for human rhino virus: TCCTCCGGCCCCTGAAT (SEQ ID NO: 998) (nt 32 to 48 of SEQ ID NO: 481).

### Reverse primers:

1^{st} set for the SarsCoV2 N gene (N1): SEQ ID NO: 496 to 591
2^{nd} set for the SarsCoV2 N gene (N3): SEQ ID NO: 592 to 687
Set for rRNA: SEQ ID NO: 688 to 783
Set for influenza A: SEQ ID NO 784 to 879
Set for influenza B: SEQ ID NO: 880 to 975
Set for human rhino virus: SEQ ID NO: 976 to 990

All reverse primers contain the same 5' part containing the adaptor sequence (GTGACTGGAGTTCAGACGTGTGCTCTTCCGATCT (SEQ ID NO: 999) - nt 1 to 34 of any of SEQ ID NO: 496 to 990); followed by the sample identifier sequence, which is different for all primers within a given amplicon-specific set, here a 8-mer for all of SEQ ID NO: 1 to 495 (e.g. AGCTTGGT (SEQ ID NO: 1000), nt 35-42 of SEQ ID NOs: 496 to 990), followed by the binding sequence for hybridization to the analyte nucleic acids; e.g. for N1: TCTGGTTACTGCCAGTTGAATCTG (SEQ ID NO: 1001) (nt 43 to 66 of SEQ ID NO: 496); for N3: tgtagcacgattgcagcattg (SEQ ID NO: 1002) (nt 43 to 63 of SEQ ID NO: 592); for rRNA: GGCAAATGCTTTCGCTCTGGTC (SEQ ID NO: 1003) (nt 43 to 64 of SEQ ID NO: 688); for influenza A; TGGACAAANCGTCTACGCTGCAG (SEQ ID NO: 1004) (nt 43 to 65 of SEQ ID NO: 784); for influenza B: GTCGCTGTTTGGAGACAC (SEQ ID NO: 1005) (nt 43 to 60 of SEQ ID NO: 880); for human rhino virus: GAAACACGGACACCCAAAGTAGT (SEQ ID NO: 1006) (nt 43 to 65 of SEQ ID NO: 976).

Staggering distancing nucleotides are only used for the forward but not the reverse primer since their effect is only needed on one side of the amplifications products to have their effects during sequencing.

### Master mix composition per reaction/well (volumes in µL)

| | |
|---|---|
| 10x PCR top up Buffer^{D} | 2.5 |
| 100 mM dUTP(NEB) | 0.07 |
| HotStart Taq Polymerase | 0.5 |
| Antarctic thermolabile UDG | 0.5 |
| Water | 16.43 |

### Thermocycler program:

10 min at 30°C (for high UDG activity)
3 min at 95°C (UDG inactivation and HotStart Taq activation)
45 cycles of: 20 sec at 95°C, 30 sec at 58°C, 20 sec at 72°C 2 min at 72°C
Cool down to 12°C (removing the plate while it's still hot will cause bending of the plastic, making further pipetting and sealing more difficult)
*^{C}PCR primer mix composition:* 2 mM of each forward and reverse primer, for all viral amplicons and 1 mM of each forward and reverse primer for the rRNA amplicon (final concentration of each primer pair in the complete 50 µL reaction is 200 and 100 nM, respectively)

### ^{D}10x PCR Top Up Buffer composition:

750 mM Tris-HCl pH 8.3
200 mM (NH₄)₂SO₄
1% Triton X-100

### Example 5: Plate pooling

All well-barcoded PCR products from a single 96-well plate were pooled, typically 20 µL of each reaction was combined in a plastic reservoir using a multi-channel pipette, and after mixing thoroughly 1 mL was transferred to an Eppendorf tube. This was repeated for every PCR plate. 5 µL from each plate pool were re-arrayed in a new 96-well plate and treated with 2 µL of illustra ExoProStar 1-step for 30 min at 37°C followed by 15 min at 80°C to remove any left-over primer.

### Example 6: Second PCR

Subset indices are added (plate barcodes) with sequencing adaptors. A master mix with all components listed below was distributed across a 96-well plate (37.5 µL/well). To each we added 10 µL of unique dual-indexed i5/i7 primer pairs (Custom synthesized index primers with Nextflex barcodes, arrayed in 96-well plates) and 2.5 µL of Exostar-treated PCR1 pool. The reactions were run for 8 cycles to add sequencing adaptors with plate barcodes.

### Master mix composition per reaction/well (volumes in µL)

| | |
|---|---|
| 10x Sequencing-ready PCR Buffer^{E} | 5 |
| 25 mM each dNTPs | 0.5 |
| 100 mM dUTP | 0.07 |
| Hot start Taq Polymerase | 0.5 |
| Water | 31.43 |

### Thermocycler program:

3 min at 95°C
8 cycles of: 15 sec at 95°C, 30 sec at 65°C, 30 sec at 72°C 2 min at 72°C
Cool down to 12°C

### ^{E}10x Sequencing-ready PCR Buffer composition:

750 mM Tris-HCl pH 8.3
200 mM (NH₄)₂SO₄
20 mM MgCl₂
0.1% Tween 20

### Example 7: Pooling and preparation for sequencing

All samples from a 96-well plate (20 µL from each well) were pooled and 250 µL of pooled sample is resolved on 2% agarose gel and 220 bp to 260 bp size amplicons are excised and gel purified using Qiagen gel extraction kit.

In order to ensure fast turnaround, the preparation of libraries for Illumina sequencing was optimized empirically. In the first four sequencing runs, standard quality control of the library, including Qubit measurement, a size analysis and qPCR, was performed. A correlation between the concentration measurement by Qubit and the qPCR was detected. In every case the molarity determined by qPCR was 10x higher than the concentration measured by Qubit. Thus, we can omit the size analysis and the qPCR, which are both time consuming. Eventually the library concentration is determined by 3 independent Qubit measurements, the obtained value in ng/µL is multiplied by 10 and used as the molarity of the sample in nanomolar. This procedure enables us to start the sequencer within a 15 min time-frame after receiving the sequencing library. Final preparation of the sequencing run happens according to Illumina's guidelines, including denaturation of the sample, neutralization and final dilution for sequencing.

### Example 8: Sequencing

Depending on the sequencer type, the following concentrations were used for sequencing: 10 pM for MiSeq V2 chemistry, 15 pM for MiSeq V3 chemistry, 2.2 pM for NextSeq550 high output and 1.3 pM for NextSeq550 medium output. In every sequencing run 10% of PhiX library were spiked-in to increase complexity. To avoid contaminations with barcodes from previous sequencing runs, the sequencers were washed with bleach according to Illumina's guidelines before every run.

### Example 9: Data analysis

The NGS data (fastq.gz files) were mapped in a single pass to sets of expected amplicon sequences and to sets of expected well- and plate indices. While any standard NGS workflow and read-mapping software (e.g. bowtie or similar) would be suitable, we used dedicated shell and awk scripts based on string-hashing that allows for 0 or 1 mismatch per amplicon and index. The i5 and i7 indices signified the plate-indices (subset index) and parts of the forward read (and the reverse read in the case of paired-end sequencing) signified the well-indices (sample index); we required the correct redundant encoding of plate and well. As the well-index on the forward read started at random offsets, we first determined the amplicon identity and position, then inferred the position of the well index, and finally compared the well index to the intended well indices.

### Example 10: Direct sample processing without RNA extraction

Many current pipelines for detection of viral RNA begin with purification of RNA from biological material (swabs, saliva, gargle, etc). However, to lower costs and match the throughput of an NGS-based pipeline, RNA extraction was bypassed. Typically, samples are collected in viral transport medium (VTM). Additionally, saliva represents an ideal viral transport medium and is now frequently used as input for SARS-CoV2 testing pipelines. We found that gargling of 10 ml HBSS is compatible with self-sampling. Gargle samples enable similar sensitivity to swabs collected by medical staff and are preferred to pure saliva as samples become more uniform in viscosity and are thus easier to pipette, making the samples better suited for automation (**Figure 1A**). Several approaches to stabilize viral RNA from respiratory specimens, circumventing RNA purification and enabling the required scalability are known (swab-seq (Bloom et al., doi.org/10.1101/2020.08.04.20167874), Salivadirect (Vogels et al., doi.org/10.1101/2020.08.03.20167791), Quick extract (Ladha et al., doi.org/10.1101/2020.05.07.20055947), HUDSON buffer (Lalli et al., doi.org/10.1101/2020.05.07.20093542)). To compare these methods, we mixed retroviral particles packaged in cell culture into gargle samples and then treated the samples according to the different protocols: i) 30 min at 95°C (swab-seq), ii) 5 min at 95 after addition of Proteinase K (Saliva-direct), iii) 1:1 mixture with QuickExtract and 5 min at 95°C, and iv) lysis and RNA extraction. We then assessed efficiency of RNA unmasking and its stability before and after treatment, by performing quantitative RT-PCR of a virus specific amplicon. We confirmed that all these methods generate stable RNA that are compatible with RT-PCR. DNA QuickExtract, however, showed the least precipitation of VTM upon boiling and was thus used for most experiments unless otherwise stated (**Figure 1B**).

### Example 11: 2-step RT-PCR has increased specificity compared to 1-step RT-PCR

The amplification of viral genome fragments for sequencing consists of RT and PCR steps. Indexes pointing towards individual samples can be incorporated during the reverse transcription (RT) step as well as during PCR (**Figure 1C**). However, we found that introduction of sample barcodes in the RT step resulted in an increased fraction of non-specific PCR products as compared to their introduction at the PCR step, presumably because the higher temperature of PCRs constitute more restrictive and thus specific conditions (**Figure 1D**). Given the large but limited sequence space on NGS flow cells, such lack of specificity means that many unspecific amplicons would be sequence, which interferes with upscaling to very large sample numbers. We therefore settled on integration of positional indexes during PCR only. Specifically, individual samples are arrayed in individual wells of 96-well plates in which the RNA from each sample is first subjected to reverse transcription (RT) and then to amplification by PCR to produce diverse desired viral and control amplicons. Priming in the RT step is performed with random hexamers as well as two gene specific twelve-mers that increase sensitivity for Sars-CoV2.

### Example 12: Compression of dynamic range by 45-cycle end-point-PCR enables sensitive detection of samples with low viral titers

One of the hurdles towards establishing a pooled NGS-based assay for samples from virus-infected individuals derives from the fact that viral loads can differ by many orders of magnitude such that high-titer samples would dominate NGS. TaqMan RT-qPCR reports differences in Ct values of 20-25 cycles, which translate into 2²⁵=33.5 million-fold differences in viral titers. Therefore, if samples with low virus titer are to be robustly identified as positives, e.g. with >100 virus-derived amplicon reads, the samples with high virus titers required 3.3x10⁹ reads, which is prohibitive. Therefore, the dynamic range needs to be compressed, in order to dampen the signal from a highly positive sample at the expense of providing sufficient sensitivity to detect samples with lower titers. In order to achieve this compression of signal, we run the first PCR reaction for 45 cycles until each individual sample reaches saturation. This results in the generation of similar numbers of amplicons per well independent of the initial viral titer (**Figure 1D****,E**). In summary, using crude respiratory specimens as input, a 2-step RT-PCR generates high specificity and even representation of characteristic amplicons across samples to enable pooling of many samples for analysis by NGS.

### Example 13: Selection of viral and human control amplicons

In addition to the very large dynamic range of viral titers between patients, nonspecific PCR amplicons can severely impact the detection of viral amplicons by NGS, because the number of NGS reads is inherently limited (and directly proportional to the total costs). For example, the parallel analysis of approximately 40K (96x384) samples means that each sample can receive a total of only ~500 reads on a MiSeq, ~2K on a HiSeq, and ~10K on a NextSeq platform. If a substantial fraction of these reads were spent on sequencing nonspecific amplicons, assay sensitivity would be severely impacted. It is thus pivotal to select amplicons and primer pairs that i) show high sensitivity ii) generate amplicons of comparable short size iii) generate few non-specific amplicons alone or in combination with any other primer pairs present in the same reaction, which is of particular importance when using primers with long extensions (here: sample-identifying barcode sequences and primer-binding sites for a 2nd PCR as discussed below).

### Example 14: 18S-rRNA-specific primer pair to control for sample presence and quality

We tested several published Sars-CoV2 specific primer pairs for the performance with our DNA overhangs. The Sars-CoV2 N-gene-specific primers N1 and N3 proposed by the Center for Disease Control (CDC), both being specific for the SARS-CoV2 N gene, performed best in SYBR-Green qPCR (which does not control for amplicon identity) as well as in initial sequencing runs, and had an ideal amplicon length of ~70. We also tested the N1 amplicon together with the widely used internal control primer pair targeting RPP30 (RNAse P). While the N1 primers showed a fair amount of correct amplicons in dependence of the amount of synthetically spiked in template, the fraction of specific amplicons for RPP30 was only 0.06-1.5% **(****Figure 2A****).** When analyzing all observed amplicons across conditions, we detected various short sequences that together used up >99% of all NGS reads. The vast majority was generated by the RPP30 specific primers **(****Fig-ure 2B****),** suggesting that these primers are not compatible with multiplexed PCR and NGS. We therefore set out to establish a new control primer pair that would produce much fewer nonspecific amplicons. We tested several primer sets on gargle samples obtained from 16 individuals, yet only a single primer pair, specific for 18S ribosomal RNA, was detected in all samples and showed a strong dependency on the presence of reverse transcriptase **(****Figure 2C****).** We therefore chose this 18S ribosomal RNA specific primer pair to control for sample quality. Of note, ribosomal amplicons were detected at Ct values of 15-45 and therefore in a comparable range as viral amplicons in patients. We therefore tested ribosomal RNA as host control in the SARSeq panel. Indeed, the ribosomal primer pair/amplicon selection performed well and neither generated abundant unspecific amplicons alone, nor in combination with N1 or N3 primer pairs **(****Figure 2E****).**

### Example 15: Synthetic spike-in RNA controls for RT-PCR performance

Since the 18S amplicon - like the RPP30 amplicon - does not span an intron it cannot discriminate against genomic DNA templates abundant in respiratory samples. In particular, QuickExtract processed gargle did not show a dominant RNA based signal even with ribosomal primers **(****Figure 2C****).** This is maybe expected since very few living cells will shed during the gargle procedure and released RNA will also not survive for extended periods of sample storage. In contrast, viral particles with genomic RNA are shed efficiently and maintained well in particular in buffered isotonic conditions such as viral transport medium (VTM), saliva, and gargle in HBSS to protect viral RNA from very abundant RNAse activity in respiratory specimen REF. To integrate an additional internal RNA control to assess successful reverse transcription in all probes, we therefore included RNA with identical primer binding sites as the ribosomal amplicon, yet unrelated sequence in between. This in vitro transcribed RNA was spiked into the reverse transcription mix at a concentration of 1000 molecules/reaction **(****Figure 2D****).** Using the RT- and PCR buffers (Examples 2, 3 and 6) SARSeq reached a very good amplicon specificity with the majority of amplicons **(****Figure 2E****).** The ratio of reads for the ribosomal amplicon and spike in serves as a very good assessment of sample quality. The high specificity of amplicons set the stage to develop a high throughput indexing strategy that allows to analyze tens of thousands of samples in parallel.

### Example 16 - comparative example: Simple sample indexing strategies don't retain sample identity

To exploit the high-throughput nature of NGS, we designed a sample barcoding strategy that allows multiplexing of tens of thousands of samples for a single sequencing run while retaining perfect sample specificity, i.e. suppressing misassignment of reads to incorrect samples. Retaining perfect sample specificity is crucial in the context of samples from healthy individuals (no viruses) and from patients with the abovementioned very wide range of virus titers. Several strategies for sample indexing are currently available that make use of the possibility that NGS can not only determine the amplicon identity but any other information on the amplicon, including sample-specific barcode sequences or indices. First, samples can be individually indexed by a sample-specific barcode in one of the two primers, i.e. in the forward or in the reverse primer **(****Figure 3A****).** In such a setup, sample-specific primers incorporate sample-indexes into the amplicons of each sample that can be detected in NGS (REF e.g. the 19000RT barcodes and swab seq 2.0). This strategy however does not scale well as it requires distinct primer pairs for each amplicon and sample (linear/additive scaling). More importantly, it cannot retain perfect sample identity due to template-switching PCR artifacts and index-hopping on flow cells, which can lead to random associations between amplicon- and index sequences. This problem is of particular relevance when high-titer samples are analyzed in parallel with samples from healthy individuals as we demonstrated by spiking synthetic Sars-CoV2 template into two wells of a 96 well plate (wells B8 and F2) in which all other wells are negative **(****Figure 3B****).** Simple combinatorial indexing with a column barcode on one primer and a row barcode on the other primer can overcome the scalability problem (swab seq 1.0) (combinatorial/multiplicative scaling; **Figure 3C****),** but suffers from the same inability to retain perfect sample identity, which in this case leads to a characteristic cross-shaped pattern along the rows and columns of the positive samples **(****Figure 3D****).**

### Example 17: Two-dimensional redundant dual indexing is scalable and retains sample identity

We implemented redundant, dual indexing (also referred to as "unique dual") that retains perfect sample identity by redundantly encoding sample identity on both ends of the amplicon, thereby eliminating illegitimate index combinations **(****Figure 3E****,F,G).** We achieve this by two indices introduced through forward and reverse primers that both point to the same sample/position (Figure 3E, G). Such an approach requires two indices (=unique primers) per sample **(****Figure 3E****)** and therefore does not scale well when a single PCR is used (1 dimension). We therefore use a two-dimensional indexing strategy by adding a second PCR step that again uses redundant, dual indexing, yet allows fully combinatorial indexing between dimension 1 and 2 (combinatorial/multiplicative; **Figure 3G****).** This strategy of two-dimensional redundant dual indexing has only a modest requirement to increase indexing primer numbers for very large panels of parallel samples and allows the encoding of 96x96 or 96x384 samples with 2x96 amplicon-specific primers (2 per amplicon; 1st dimension) plus 2x96 or 2x384 global primers (irrespective of amplicon; 2nd dimension), respectively, when using standard 96- and 384-well plates, for example. Greater number of index-es/primers are easily scalable due to the multiplicity of the 2-step index strategy, which presents a robust and scalable indexing strategy.

### Example 18: A set of 96 validated primer pairs per amplicon for the 1st dimension

In practice, we extended the amplicon-specific primers for the 1st PCR (1st dimension - sample indexing) at their 5' ends to include a sample-specific barcode and i5/i7 sequences as primer-binding sites for the 2nd PCR. To ensure sufficiently complex sequences of the NGS forward reads for cluster identification and stable sequencing, we staggered the sample-barcode and the amplicon-specific sequence by a random offset of 1-4 base pairs in the forward primers - see "n" nucleotides in SEQ ID NO: 1 to 495 **(see** **Figure 3G** **and Example 4).** We tested a total of 110 primer pairs per amplicon to establish a set of 96 primer pairs that show good amplification behavior for all amplicons **(****Figure 7****).** Importantly, all amplicons within one well obtain the identical stagger and index to also prevent recombination between amplicons during PCR. Pre-prepared primer-plates and robotic pipetting pipelines allow us to process thousands of samples in parallel.

### Example 19: Perfectly retaining sample identity across the two dimensions

After the indexing of individual samples (=wells of a 96-well plate; 1st dimension), all samples of one plate are pooled to one position in a new 96-well plate, and in a second PCR, a plate specific index is added (2nd dimension). We implemented four measures to ensure that sample identity is perfectly retained between the 1st and 2nd PCR. i) To avoid that primers from the 1st PCR are present during the 2nd PCR, we included an RNA template with N1 and N3 primer binding sites similar to our RT control and the denominator used in the Swab-Seq pipeline to suppress stochastic contaminations. In addition, we ii) treated the pools with DNA exonuclease to enzymatically degrade all single-stranded DNA and thus all remaining primers especially from Sars-CoV2 negative wells. iii) We keep the cycle number for PCR 2 at a minimum to avoid amplicon recombination during PCR and use a PCR protocol that prevents premature termination of an extension step. Indeed, the measures synergistically contributed to the robustness of read assignment **(****Fig. 8A****).** iv) In each dimension we use dual and redundant indices with a Lowenstein distance of at least three mismatches. This pipeline also suppressed read misassignment across plates, such that the positions of Figure 3A, C, E were not detected on other plates run in the same experiment **(****Fig. 8B****).**

We thus established a scalable and robust indexing for Sars-CoV2 by setting up a PCR-based indexing-strategy, that incorporates two sets of redundant indices and we bioinformatically filter to only allow legitimate combinations of those four indices. The ability to encode 96 well barcodes and 384 plate barcodes means this can be used to investigate 36,864 individual samples simultaneously. To illustrate scalability of the approach, a 4-fold increase in each dimension for example by using 384-well plates would enable multiplexing of >145,000 samples. Sequencing capacity is thus no longer a bottleneck to frequent population wide testing for many countries and the sequencing price is negligible. This indexing systems allows to filter against any DNA remnants in sequencing flow cells, that can result in read misassignment between independent analysis runs. In summary, dual redundant indexing introduced during PCR 1 ensures sample identity specificity and two-dimensional indexing allows scalability while preventing any spill of reads from positive to negative samples even across multiple orders of magnitude in signal intensity. We termed this direct analysis of respiratory samples using next generation sequencing SARSeq for "Saliva Analysis by RNA Sequencing".

### Example 20: Specificity and Sensitivity of SARSeq

To test sensitivity, specificity, and scalability of SARSeq we set out to run large sample cohorts. To assay under close to real-life conditions we prepared samples plates and processed them in parallel using a robotic pipetting platform. We also tested the effect of spike-ins with identical primer binding sites to the N1/N3 amplicons but different sequences in between, as introduced previously.

Initially we tested sensitivity of the SARSeq pipeline by diluting viral templates to 1, 3, or 10 copies per 5ul probe volume (2-0.2 copies/ul). To account for the contribution of Quick Extract as input buffer to the pipeline as well as to test RNA extraction with this method we tested above dilutions with synthetic RNA in H₂O as well as Quick Extract mixed to HBSS and also assayed viral particles in Quick extract. Using H₂O for dilution we were able to detect Sars-CoV2 in 5 and 6 of 24 tested cases for N1 and N3 respectively at a dilution of in average 1 amplicon per well **(****Figure 4A****).** At such dilution 63% of wells are expected to contain one or more viral templates assuming a Pois-son distribution pointing towards a detection efficiency of 0.3-0.4 per molecule. Quick Extract increased that efficiency to 0.7 while detection straight from viral particles was at 1.1 per molecule **(****Figure 4B****).** Presence of N1 and N3 denominator spike-ins even showed slightly better detection efficiencies. It is assumed that infectious Covid-19 patients show with detectable viral titers of >10³/µL. Our detection limit is thus at least 100 times more sensitive than required for mitigation of the current Sars-CoV2 pandemic. Next, we tested the effect of spike-ins on the quantitative behavior of SARSeq **(****Figure 4C****).** Due to the endpoint PCR we perform, SARSeq intentionally only returns semiquantitative results. However, when testing a dilution series of synthetic spikes in quadruplicate we noticed an improved reproducibility in presence of these spike-ins and therefore included them in the final setup.

To challenge SARSeq with hundreds of real samples omitting RNA purification, we next generated sample plates from gargle samples collected in HBSS. Such crude samples may contain reagents inhibitory to the RT or PCR step. To this end, we generated a dilution series of synthetic template and also spiked a dilution series of a subject or patient sample presenting with Ct=30 in a taqman Q-PCR assay into several positions. Indeed, when testing six 96-well plates, we were able to detect all patients previously detected with an RNA purification followed by a Taqman-based qPCR. In addition, we saw all patients and synthetic spike-ins that we had added prior to running the pipeline. Overlap between N1 and N3 amplicon-based results was 100% **(****Figure 4D****).**

Another critical parameter when testing very large numbers of patients is the false positive rate. We were therefore extremely pleased to see that our indexing strategy and pipeline delivered typically zero and very rarely 1 read indicative of SARS-CoV2 for gargle of persons previously tested negative by Q-PCR as well as all H₂O controls. This binary result showcased an unambiguous assessment of infection status by SARSeq. Due to the absence of false positive results we did not need to use our denominator amplicons to set a threshold for signal strength for positive results. However, we envision that they could be a powerful method to reduce stochastic reads if incorporated at sufficiently high concentration to reduce sensitivity.

### Examle 21: Performance of SARSeq on large panels of patient samples

To test if SARSeq would robustly detect real SARS-CoV2 virus in VTM samples collected in clinical diagnostic pipelines of many independent patients, we tested a set of 564 samples with SARSeq in duplicate. While we did not detect N1 or N3 amplicons in H₂O or -RT conditions **(****Figure 5A, B****)** we frequently obtained SarsCoV2 reads across all plates with good correspondence between amplicons and replicates. **(****Figure 5C, D****).** To assay correspondence to the standard test, we also measured the samples in a Taqman Q-PCR assay in duplicates. Expectedly we found a very good correspondence of both replicates for Ct values below 36 **(****Figure 5E****, red dots)** and a stochastic behavior at the detection border **(****Figure 5E****, orange dots)** with either one or two replicates scoring positive. No Sars-CoV2 was detected in an additional 354 samples. We analyzed the overlap between detection in qPCR- and NGS-based assays and found that 157 or 96.3% of samples that scored with a Ct value of <36 in at least one qPCR in fact scored positive in all four assays while six samples were detected with one or two assays only **(****Figure 5F****).** We hypothesized that the stochastic behavior at detection border is due to presence or absence of a single viral genome in the reaction. If that was true, then correspondence between amplicons of a single replicate is expected to be better than correspondence per amplicon between replicates even though N1 and N3 might show different sensitivities. Indeed, of the samples detected with 1-3 assays, 48 and 43 samples show detection of Sars-CoV2 with both amplicons within one replicate while only 18 and 25 samples are detected twice independently with N1 or N3, respectively. We thus conclude that SARSeq is sensitive down to single viral genomes. We plotted read counts of both SARSeq replicates against each other to visualize read count reproducibility **(****Figure 5G, H****).** As anticipated, we see a lower correlation than with qPCR, some samples undetected in NGS but present in one qPCR run at any Ct, as well as the detection of some samples missed by one or both Q-PCRs. We also observe a distinct upper end of N1/N3 read counts attributed to the intentional blunting of reads in end point PCR to distribute sequencing space evenly across samples (see also Figure 1E). Taken together, SARSeq was able to reproducibly identify Sars-CoV2 across a patient cohort of 564 samples with high reproducibility and a sensitivity to single molecule level.

### Example 22: Broader applicability to detection of respiratory infectious agents

Multiple infectious agents cause disease with overlapping clinical symptoms, foremost Influenza A and B. Moreover, it is expected that particularly in the winter season, various respiratory symptoms will cause concerns for patients (even if clinical symptoms are not strictly overlapping) and thereby dramatically increase the demand for reliable/sensitive Sars-CoV2 tests. Most prominently, we anticipate that rhinoviral infections will remain frequent even in measures of social distancing as a typical winter season brings several episodes of rhinoviral infection per person. For SARSeq additional amplicons come at no extra cost if it doesn't increase sequencing depth requirements. Therefore, we can further multiplex SARSeq to detect in addition other common respiratory viruses found in the same type of sample as SARS-CoV2.

To accommodate for the aforementioned RNA viruses, we sought to optimize primers for influenza A, B, and rhinovirus to be combined with our Sars-CoV2 specific SARSeq pipeline. To this end we selected primers based on qPCR performance, amplicon length, and an NGS pilot. For a pan-influenza A amplicon we settled on combining a degenerated forward primer from Bose et al. (J Clin Microbiol 47(9), 2009: 2779-2786) with a degenerate WHO reverse primer, both targeting the M gene. For pan-influenza B we selected a primers (see example 4) of SEQ ID NO: SEQ ID NO: 385 to 480 (forward primers) and SEQ ID NO: 880 to 975 (reverse primers) to the M gene. Rhinovirus is detected using a primer pair of Do et al. (J Mol Diagn. 2010; 12(1): 102-108).

To test performance across a large number of specimen we took gargle-Quick extract samples and spiked in purified RNA obtained from HEK293T cells infected with respective virus strains 48 h prior purification at a voluminometric ratio of 1:100 or dilutions thereof. Samples were processed using the robotic pipeline. We performed PCR1 in the presence of six primer pairs, two against Sars-CoV2, and one each for ribosomal control, influenza A, influenza B, and rhinovirus. Upon pooling and PCR2, samples were sequenced by paired end sequencing to account for the presence of longer amplicons, and reads were mapped to wells/samples **(****Figure 6B****).** Of note, by pipetting spike-in RNA prior to setting up the RT reaction we apparently generated an RNA contamination of influenza B that was stochastically distributed across all reactions (including H₂O controls but not -RT controls) and obtained reproducibly lower read counts than spike in samples. This event emphasizes the importance of a local separation of reaction mix preparation and sample handling. All post-PCR steps were always performed in a different lab from the beginning and thus DNA amplicons did not generate detectable contamination of our pipeline. Nevertheless, we implemented incorporation of UTP and a UDG digestion step prior to PCR in our protocol to protect against contamination risk with amplicons. Importantly, however, our multiplexes pipeline to detect RNA of various viral respiratory diseases in parallel performed robustly across six 96-well plates. We obtained no false negative samples besides expectedly the 1/1 mio dilutions of a Sars-CoV2 sample with Ct30. Aside from the apparent influenza B sample contamination, we did also not see any other false positive events. Moreover, the amplicon sequence of influenza A allowed us to distinguish between the two spike-in strains we had used, namely A/WSN and A/Wy. This is of particular interest since A/Wy like B/Lee used as an Influenza B spike-in is contained in the current vaccination as it is anticipated to circulate in the northern hemisphere 2020/21 flu season. Similarly, we were able to distinguish between the three rhinoviral strains used as spike-in, namely HRV A1a, A1b and A2, by polymorphism in respective amplicons. Taken together our pipeline in its current setup thus differentially detects seven different viral respiratory agents in a single reaction, contains various internal controls, a sample quality control, and by design has a particular sensitivity for Sars-CoV2. SARSeq thus represents a multiplexed, massively parallelized assay for the detection of respiratory infections by means of RT-PCR and next generation sequencing.

### Example 23: Sars-CoV2 Spike variant detection

A variation of the method described in examples 2-9 allows sensitive detection of commonly known and new variants of the spike protein of Sars-CoV2 on a large scale. This method is employed to routinely analyze 2400 per day in high throughput.

The method basically follows example 2-9 with minor modifications in the methodology, using different primers, however, to analyze different parts of the spike protein. In particular, 13 different amplicons for the spike protein are generated. These cover the nucleic acid sequences corresponding to amino acids 1-722 and 767-839 of the spike protein, which is where the immunologically relevant mutations are found.

Briefly, the method contains the steps:
**1. RNA extraction:** For illustration purposes, the method is exemplified using purified RNA. However, it also works with other sample types, such as gargle or saliva, as described in example 1.
**2. Reverse transcription:** RT is done as described as in example 2 with modified primers. Two separate parallel reactions are performed per sample, each with distinct sets of RT primers ("odd" and "even"). The method and spike gene coverage by the primer sets are illustrated in Figure 9. Briefly, 5 µl of sample are added to each an "odd" and an "even" complete RT mix (20 µ1 per reaction). Plates are sealed and incubated at 55°C for 30 min and inactivated 3 min at 95°C. The primers have been selected for specificity of different parts of the S gene (see Figure 9C) and for having a melting temperature of about 58°C. Selecting a melting temperature slightly above the working temperature of the reverse transcriptase (55°C) avoids efficiency loss due to secondary structure of the virus genome.
   The RT primer sequences are:
   1: CATGTATAGCATGGAACCAAGTAACA (SEQ ID NO: 1007)
   2: CGTTATTAACAATAAGTAGGGACTGGGT (SEQ ID NO: 1008)
   3: CCATCAATATTCTTAAACACAAATTCCCTAAG (SEQ ID NO: 1009)
   4: CCCACATAATAAGCTGCAGCA (SEQ ID NO: 1010)
   5: CTACAGTGAAGGATTTCAACGTACA (SEQ ID NO: 1011)
   6: GCATAGACATTAGTAAAGCAGAGATCA (SEQ ID NO: 1012)
   7: CAGGTAATTATAATTACCACCAACCTT (SEQ ID NO: 1013)
   8: AGTTCAAAAGAAAGTACTACTACTCTGT (SEQ ID NO: 1014)
   9: CCAAATTGTTGGAAAGGCAGAAAC (SEQ ID NO: 1015)
   10: GCATGAATAGCAACAGGGACT (SEQ ID NO: 1016)
   11: GTCTGATAACTAGCGCATATACCTG (SEQ ID NO: 1017)
   12: GTACAATCTACTGATGTCTTGGTCA (SEQ ID NO: 1018)
   13: GTGCACAAATGAGGTCTCTAGCA (SEQ ID NO: 1019)
   7 (alternative version): GAGATTAGACTTCCTAAACAATCTATACAGGT (SEQ ID NO: 1020)
**3. PCR1:** A first PCR reaction is performed similar to example 4. cDNA plates are opened and 25 µl of complete PCR top up mix (including primers) is added. An "even" PCR mix is added to the even numbered RT plates and an "odd" PCR mix is added to the odd numbered RT plates. The PCR primers in addition to being specific for the 13 different S gene fragments ("tiles") (Fig. C) have the same structure as the primers described in example 4, i.e. they have unique dual index combinations in the forward and the reverse primer that point to a specific well in a plate (see Figure 3). While in example 4 a 96 distinct dual index combination was used, here 12 or 24 indices are used for cost considerations, but conceptually the same principles as described in example 4 are adhered to, the only difference is that in the next step not a complete plate is pooled, but only 12 (or 24) samples that carry different indices are pooled together.

As described in example 4, the forward primers again contain a 5' part containing the adaptor sequence, followed by the staggering nucleotides, i.e. 1 to 4 nucleotides, which are randomly selected from A, C, G, or T, followed by the sample identifier sequence, which is different for all primers within a given amplicon-specific set, e.g. an 8-mer, followed by a binding sequence for hybridization to the analyte nucleic acids.

Also as described in example 4, all reverse primers contain the same 5' part containing the adaptor sequence, followed by the sample identifier sequence, which is different for all primers within a given amplicon-specific set, e.g. an 8-mer, followed by the binding sequence for hybridization to the analyte nucleic acids.

The binding sequence for hybridization to the analyte nucleic acids for the spike gene fragments are:
1 - forward: GGGGTACTGCTGTTATGTCTTTAAAAGA (SEQ ID NO: 1021)
1 - reverse: CTGAGGATCTGAAAACTTTGTCAGGGT (SEQ ID NO: 1022)
2 - forward: CCCCTGCATACACTAATTCTTTCACAC (SEQ ID NO: 1023)
2 - reverse: CTGGGTCTTCGAATCTAAAGTAGTACCA (SEQ ID NO: 1024)
3 - forward: GCTTCCACTGAGAAGTCTAACATAATAAGAGG (SEQ ID NO: 1025)
3 - reverse: CCCTGTTTTCCTTCAAGGTCCA (SEQ ID NO: 1026)
4 - forward: GTTGGATGGAAAGTGAGTTCAGAGT (SEQ ID NO: 1027)
4 - reverse: ACCTATTGGCAAATCTACCAATGGTTC (SEQ ID NO: 1028)
5 - forward: GCGTGATCTCCCTCAGGGTTT (SEQ ID NO: 1029)
5 - reverse: CGTACACTTTGTTTCTGAGAGAGGGT (SEQ ID NO: 1030)
6 - forward: CAGATGCTGTAGACTGTGCACTT (SEQ ID NO: 1031)
6 - reverse: CACTTAAAAGTGGAAAATGATGCGGAA (SEQ ID NO: 1032)
7 - forward: GGAAGAGAATCAGCAACTGTGTTGC (SEQ ID NO: 1033)
7 - reverse: CACCAACCTTAGAATCAAGATTGTTAGAATTCC (SEQ ID NO: 1034)
8 - forward: CCAGATGATTTTACAGGCTGCGT (SEQ ID NO: 1035)
8 - reverse: GTACTACTACTCTGTATGGTTGGTAACCAAC (SEQ ID NO: 1036)
9 - forward: GTCTAATCTCAAACCTTTTGAGAGAGAT (SEQ ID NO: 1037)
9 - reverse: GAACACCTGTGCCTGTTAAACCAT (SEQ ID NO: 1038)
10 - forward: GCACCAGCAACTGTTTGTGGAC (SEQ ID NO: 1039)
10 - reverse: CAGCAACCTGGTTAGAAGTATTTGTTCC (SEQ ID NO: 1040)
11 - forward: CGTGATCCACAGACACTTGAGATTCT (SEQ ID NO: 1041)
11 - reverse: CCTGCACCAATGGGTATGTCACA (SEQ ID NO: 1042)
12 - forward: GCAGGCTGTTTAATAGGGGCTG (SEQ ID NO: 1043)
12 - reverse: GGTCATAGACACTGGTAGAATTTCTGTGG (SEQ ID NO: 1044)
13 - forward: GGCAGTTTTTGTACACAATTAAACCGTG (SEQ ID NO: 1045)
13 - reverse: TCTAGCAGCAATATCACCAAGGCA (SEQ ID NO: 1046)

In this example 24 different primers for each group with different sample identifier sequences, which are different for all primers within a given amplicon-specific set, e.g. 24 "1- forward", 24 "1- reverse", etc., primers, are used for different wells as described for example 4.

**4. PCR2:** Samples are pooled by plate (if using 96 indices) or by row (if using 12 indices only) and each pool is first treated with Exostar to degrade left-over primers and nucleotides, and subsequently subjected to a second PCR as described in example 7, adding a "plate" or "row" index that allows combinatorial encoding of samples and therefore higher throughput. PCR2 primers also include the Illumina flow-cell adaptors as described in example 7.

**5. Library clean-up:** PCR2 produces sequencing-ready products that simply need to be pooled and cleaned up by gel purification to remove primer dimers and unspecific smaller amplicons.

**6. Sequencing:** Illumina sequencing according to manufacturer instructions is performed. More sequencing depth is used than in example 2-9 because longer regions are covered.

Data analysis shows a high coverage of all amplicons (Figure 10). In a practical application, 2200-2500 samples from Covid19 patients were analyzed each week. Figure 11 shows the detected SARS-CoV2 variants with amino acids (aa) changes as compared to the normal variant. Figure 12 shows the changes in SARS-CoV2 variants detected per week (calendar weeks 3-8 of the year 2021 are shown).

### Summary

The global pandemic caused by Sars-CoV2 has majorly impacted on our societies and economies worldwide. It has demonstrated that most countries are not prepared to fight such challenges despite our progress in modern medicine as well as our conceptual understanding of infection cascades. Social distancing measures to interfere with further spread of Sars-CoV2 are likewise affecting almost all aspects of human life. To minimize such measures, it is required to implement them as directed as possible, for which data of infection events are needed. Importantly, such screening efforts serve a different goal than testing symptomatic individuals for Sars-CoV2. While the latter requires gold-standard methods to advise on the optimal treatment, screening for mitigation does allow for some ambiguity especially if the alternative is not to test. However, mass testing for mitigation comes with major practical challenges: i) Sample collection requires major investment into logistics. Amongst them are supply chain issues, personnel, the need for digital data management, and often legal hurdles. Also, communication to improve compliance has been identified as an increasing challenge. ii) Tests must be highly specific for Sars-CoV2 and almost devoid of false positives to prevent isolation of people based on erroneous results to build trust. NGS based detection of Sars-CoV2 can supply such results by detecting genome fragments at a precision even more specific than Taqman probes can be. In addition, the detection of two fragments in parallel represents an independent detection. iii) Costs for mass testing must be as low as possible to reasonably enable scaling. SARSeq as presented here relies on enzymes that can be purchased at scale and produced in-house. Buffers are made from standard salts. iv) Mass testing must not interfere or be confused with the tests performed in medical/diagnostic facilities. It should thus neither be carried out at the same facilities nor compete for supply required to diagnose symptomatic patients. SARSeq relies exclusively on equipment available in sufficient quantity in classical molecular biology facilities such as universities, institutes, biotech, and pharma. v) Tests must scale massively. We have set up SARSeq for a maximum analysis of 36K samples in parallel and tested up to 20K samples in a single sequencing run. Therefore, NGS capacities do not represent a practical limitation of this method. SARSeq is limited by sample supply as well as the throughput of PCRs that can be implemented.

Beyond Sars-CoV2, the pipeline can be rapidly adapted to additional amplicons for the detection of other infectious agents such as newly emerging viruses. We already implemented parallelized screening for Sars-CoV2, Influenza A and B, as well as HRV and aim to extend it to further respiratory infections with overlapping clinical manifestations such as RSV. Moreover, any newly emerging virus can be added to the panel. Also, SARSeq is not limited to respiratory specimen but the pipeline can be extended to other human samples or even monitoring pipelines such as waste-water monitoring.

A limitation of SARSeq is the time requirement of the assay. Two PCR reactions must be performed followed by sequencing (NGS) and analysis, so the theoretical time requirement is around 15h. Therefore, SARSeq is not ideally suited for situations where immediate results are required. In such cases, antigen tests or RT-LAMP are superior methods. Rather, it is ideally implemented for regular (e.g. weekly) surveillance of infections at very large scale. Also, SARSeq is currently only semiquantitative. If exact viral titers are required e.g. to terminate quarantine it might be preferable to use non-multiplexed qPCR.

## Claims

1. A method for detecting a nucleic acid of interest in a plurality of samples, comprising the steps of
a) providing analyte nucleic acids from a plurality of samples in separate containers for each sample, wherein the containers are arranged into an array of subsets, wherein the array comprises two or more subsets;
b) amplifying nucleic acids by a primer extension reaction using at least one pair of primers hybridized to the analyte nucleic acids, wherein a pair of primers comprises a forward and a reverse primer, wherein the forward and reverse primers both comprise in 5' to 3' direction an adaptor sequence, a sample identifier sequence and a binding sequence for hybridization to the analyte nucleic acids, respectively, and wherein in the forward primer the sample identifier sequence and the binding sequence for hybridization to the analyte nucleic acids are staggered by a random offset of 1-4 bases;
c) combining the amplified nucleic acids of step b) of containers of two or more subsets to an array of combined containers, wherein containers of one subset, but not of another subset, are combined to a combined container;
d) amplifying nucleic acids by a primer extension reaction using at least one pair of further primers hybridized to the amplified nucleic acids of combined containers of step c), wherein a pair of further primers comprises a further forward and a further reverse primer, wherein the further forward and further reverse primers both comprise a subset identifier sequence and a sequence for hybridization to the adaptor sequence;
e) determining the sequences of the amplified nucleic acids of step d);
f) assigning a determined sequence of a nucleic acid of interest of step e) to a sample through association to a subset and container with the subset identifier sequences and the sample identifier sequences.

2. The method of claim 1, wherein i) in step b) each sample identifier sequence of the forward primers is different from sample identifier sequence of other forward primers for a given binding sequence for hybridization to the analyte nucleic acids; and/or
ii) in step b) each sample identifier sequence of the reverse primers is different from sample identifier sequence of other reverse primers for a given binding sequence for hybridization to the analyte nucleic acids;
and/or
iii) in step d) each subset identifier sequence of the further forward primers is different from subset identifier sequence of other further forward primers;
and/or
iv) in step d) each subset identifier sequence of the further reverse primers is different from subset identifier sequence of other further reverse primers;
preferably a combination of i), ii), iii) and iv).

3. The method of claim 1 or 2, wherein the further primers of step d) comprise a sequencing adaptor sequence.

4. The method of any one of claims 1 to 3, wherein the step a) comprises providing a RNA from a sample and generating a cDNA of said RNA by reverse transcription, wherein the nucleic acids that are amplified in step b) comprise said cDNA.

5. The method of any one of claims 1 to 4, wherein the at least one of the forward and reverse primers of step b) is depleted during and/or after step b) but before step c).

6. The method of claim 5, wherein the depletion comprises amplification of a spike-in nucleic acid with the forward and/or reverse primer during step b).

7. The method of claim 5 or 6, wherein the depletion of the forward and reverse primers comprises treatment with a single-strand specific nuclease.

8. The method of any one of claims 1 to 7, wherein amplifying nucleic acids in step d) is restricted to at most 35 amplification cycles, preferably at most 20 amplification cycles.

9. The method of any one of claims 1 to 8, wherein the plurality of samples comprises 4000 or more samples and/or the two or more subsets are 40 or more subsets and/or wherein a subset comprises 40 or more containers.

10. The method of any one of claims 1 to 9, wherein the nucleic acid of interest is a pathogen nucleic acid, preferably a viral nucleic acid.

11. The method of claim 10, wherein the nucleic acid of interest is selected from a coronavirus nucleic acid, preferably a SARS-CoV-2 nucleic acid, an influenza nucleic acid, a parainfluenza nucleic acid, a rhinovirus nucleic acid, or a combination thereof.

12. The method of any one of claims 1 to 11, wherein two or more nucleic acids of interest are selected, preferably wherein the two or more nucleic acids of interest are from a virus, which is the same virus or a different virus for two or more nucleic acids of interest, and/or preferably wherein in step b) forward and reverse primers with binding sequences for hybridization to the two or more analyte nucleic acids are used.

13. The method of any one of claims 1 to 12, wherein the sample identifier sequence of the forward primers, the sample identifier sequence of the reverse primers, the subset identifier sequence of the further forward primers, and the subset identifier sequence of the further reverse primers, respectively, comprise a sequence distance, preferably a Hamming distance or a Levenshtein distance, to other sample identifier sequences of forward primers, sample identifier sequences of the reverse primers, subset identifier sequences of further forward primers, and subset identifier sequences of further reverse primers, respectively, of at least 2, preferably of at least 3.

14. The method of any one of claims 1 to 13, wherein the sample identifier sequence of the forward primers, the sample identifier sequence of the reverse primers, the subset identifier sequence of the further forward primers, and the subset identifier sequence of the further reverse primers, respectively, each selected independently, have a length of at least 4 nucleotides.

15. A set of primers suitable for a method of any one of claims 1 to 14, comprising
at least 10 different primers A which comprise the sequence, from 5' to 3': an adaptor A sequence, an identifier sequence of at least 4 nt in length and a target binding sequence, wherein the identifier sequence is different within the at least 10 different primers A, preferably with a sequence distance of a Hamming distance of at least 1 or a Levenshtein distance of at least 1, and wherein the sample identifier sequence and the binding sequence for hybridization to the analyte nucleic acids are staggered by a random offset of 1-4 bases;
at least 10 different primers B which comprise the sequence, from 5' to 3': an adaptor B sequence, an identifier sequence of at least 4 nt in length and a target binding sequence, wherein the identifier sequence is different within the at least 10 different primers B, preferably with a sequence distance of a Hamming distance of at least 1 or a Levenshtein distance of at least 1;
at least 10 different primers C which comprise the sequence, from 5' to 3': an adaptor C sequence, an identifier sequence of at least 4 nt in length and a binding sequence that binds to the adaptor A sequence, wherein the identifier sequence is different within the at least 10 different primers C, preferably with a sequence distance of a Hamming distance of at least 1 or a Levenshtein distance of at least 1; and
at least 10 different primers D which comprise the sequence, from 5' to 3': an adaptor D sequence, an identifier sequence of at least 4 nt in length and a binding sequence that binds to the adaptor B sequence, wherein the identifier sequence is different within the at least 10 different primers D, preferably with a sequence distance of a Hamming distance of at least 1 or a Levenshtein distance of at least 1.

16. The set of primers according to claim 15, wherein the target binding sequences of the primers A and primers B comprise a sequence or antisense-sequence, respectively, of a viral gene, preferably of a SARS-CoV-2 N, M, E or S gene or coding sequence thereof, Influenza PA, PB1, PB2, PA, HA, NP, NA, M1, M2, NS1 or NEP gene or coding sequence thereof, a parainfluenza HN, F, M, NP, P or L gene or coding sequence thereof, a rhinovirus VP1, VP2, VP3, VP4, 2A, 2B, 2C, 3A, 3B, 3C or 3D gene or coding sequence; a human respiratory syncytial virus A (HRSV-A) GA1, GA2, GA3, GA4, GA5, GA6, GA7, SAA1, NA1, or NA2 gene or coding sequence; a human respiratory syncytial virus B (HRSV-B) GB1, GA2, GA3, GB4, SAB1, SAB2, SAB3, BA1, BA2, BA3, BA4, BA5, or BA6 gene or coding sequence.

17. The set of primers according to claim 15 or 16, further comprising a primer pair that binds to a ribosomal gene or coding sequence.

## Patentansprüche

1. Verfahren zum Nachweis einer Nukleinsäure von Interesse in einer Vielzahl von Proben, umfassend die Schritte
a) Bereitstellen von Analyten-Nukleinsäuren aus einer Vielzahl von Proben in separaten Behältern für jede Probe, wobei die Behälter in einer Anordnung von Untergruppen angeordnet sind, wobei die Anordnung zwei oder mehr Untergruppen umfasst;
b) Amplifizieren von Nukleinsäuren durch eine Primer-Verlängerungs-Reaktion unter Verwendung mindestens eines Paares von Primern, die mit den Analyten-Nukleinsäuren hybridisiert sind, wobei ein Paar von Primern einen Vorwärts- und einen Rückwärts-Primer umfasst, wobei die Vorwärts- und Rückwärts-Primer beide jeweils in 5'- bis 3'-Richtung eine Adapter-Sequenz, eine Probenidentifizierungs-Sequenz und eine Bindungssequenz zur Hybridisierung mit den Analyten-Nukleinsäuren umfassen, und wobei im Vorwärts-Primer die Probenidentifizierungs-Sequenz und die Bindungssequenz zur Hybridisierung mit den Analyten-Nukleinsäuren um einen zufälligen Versatz von 1-4 Basen versetzt sind;
c) Kombinieren der amplifizierten Nukleinsäuren aus Schritt b) von Behältern von zwei oder mehr Untergruppen zu einer Anordnung von kombinierten Behältern, wobei Behälter einer Untergruppe, aber nicht einer anderen Untergruppe, zu einem kombinierten Behälter kombiniert werden;
d) Amplifizieren von Nukleinsäuren durch eine Primer-Verlängerungs-Reaktion unter Verwendung von mindestens einem Paar weiterer Primer, die mit den amplifizierten Nukleinsäuren der kombinierten Behälter von Schritt c) hybridisiert sind, wobei ein Paar weiterer Primer einen weiteren Vorwärts- und einen weiteren Rückwärts-Primer umfasst, wobei die weiteren Vorwärts- und die weiteren Rückwärts-Primer beide eine Untergruppen-Identifizierungs-Sequenz und eine Sequenz zur Hybridisierung mit der Adapter-Sequenz umfassen;
e) Bestimmen der Sequenzen der amplifizierten Nukleinsäuren aus Schritt d) ;
f) Zuordnen einer bestimmten Sequenz einer Nukleinsäure von Interesse aus Schritt e) zu einer Probe durch Assoziation zu einer Untergruppe und einem Behälter mit den Untergruppen-Identifizierungs-Sequenzen und den Probenidentifizierungs-Sequenzen.

2. Verfahren nach Anspruch 1, wobei i) in Schritt b) jede Probenidentifizierungs-Sequenz der Vorwärts-Primer sich von der Probenidentifizierungs-Sequenz anderer Vorwärts-Primer für eine gegebene Bindungssequenz zur Hybridisierung mit den Analyten-Nukleinsäuren unterscheidet; und/oder
ii) in Schritt b) jede Probenidentifizierungs-Sequenz der Rückwärts-Primer sich von der Probenidentifizierungs-Sequenz anderer Rückwärts-Primer für eine gegebene Bindungssequenz zur Hybridisierung mit den Analyten-Nukleinsäuren unterscheidet;
und/oder
iii) in Schritt d) jede Untergruppen-Identifizierungssequenz der weiteren Vorwärts-Primer sich von der Untergruppen-Identifizierungssequenz anderer weiterer Vorwärts-Primer unterscheidet;
und/oder
iv) in Schritt d) jede Untergruppen-Identifizierungs-Sequenz der weiteren Rückwärts-Primer sich von der Untergruppen-Identifizierungssequenz anderer weiterer Rückwärts-Primer unterscheidet;
vorzugsweise eine Kombination von i), ii), iii) und iv).

3. Verfahren nach Anspruch 1 oder 2, wobei die weiteren Primer von Schritt d) eine Sequenzierungs-Adapter-Sequenz umfassen.

4. Verfahren nach einem der Ansprüche 1 bis 3, wobei der Schritt a) das Bereitstellen einer RNA aus einer Probe und das Erzeugen einer cDNA dieser RNA durch Reverse Transkription umfasst, wobei die Nukleinsäuren, die in Schritt b) amplifiziert werden, diese cDNA umfassen.

5. Verfahren nach einem der Ansprüche 1 bis 4, wobei der mindestens eine der Vorwärts- und Rückwärts-Primer von Schritt b) während und/oder nach Schritt b), aber vor Schritt c), abgereichert wird.

6. Verfahren nach Anspruch 5, wobei die Abreicherung die Amplifikation einer Spike-in-Nukleinsäure mit dem Vorwärts- und/oder Rückwärts-Primer während Schritt b) umfasst.

7. Verfahren nach Anspruch 5 oder 6, wobei die Abreicherung der Vorwärts- und Rückwärts-Primer eine Behandlung mit einer einzelstrangspezifischen Nuklease umfasst.

8. Verfahren nach einem der Ansprüche 1 bis 7, wobei das Amplifizieren von Nukleinsäuren in Schritt d) auf höchstens 35 Amplifikationszyklen, vorzugsweise auf höchstens 20 Amplifikationszyklen, beschränkt ist.

9. Verfahren nach einem der Ansprüche 1 bis 8, wobei die Vielzahl der Proben 4000 oder mehr Proben umfasst und/oder die zwei oder mehr Untergruppen 40 oder mehr Untergruppen sind und/oder wobei eine Untergruppe 40 oder mehr Behälter umfasst.

10. Verfahren nach einem der Ansprüche 1 bis 9, wobei die Nukleinsäure von Interesse eine pathogene Nukleinsäure, vorzugsweise eine virale Nukleinsäure, ist.

11. Verfahren nach Anspruch 10, wobei die Nukleinsäure von Interesse ausgewählt ist aus einer Coronavirus-Nukleinsäure, vorzugsweise einer SARS-CoV-2-Nukleinsäure, einer Influenza-Nukleinsäure, einer Parainfluenza-Nukleinsäure, einer Rhinovirus-Nukleinsäure oder einer Kombination davon.

12. Verfahren nach einem der Ansprüche 1 bis 11, wobei zwei oder mehr Nukleinsäuren von Interesse ausgewählt werden, vorzugsweise wobei die zwei oder mehr Nukleinsäuren von Interesse von einem Virus stammen, das das gleiche Virus oder ein anderes Virus für zwei oder mehr Nukleinsäuren von Interesse ist, und/oder vorzugsweise wobei in Schritt b) Vorwärts- und Rückwärts-Primer mit Bindungssequenzen zur Hybridisierung an die zwei oder mehr Analyten-Nukleinsäuren verwendet werden.

13. Verfahren nach einem der Ansprüche 1 bis 12, wobei die Probenidentifizierungs-Sequenz der Vorwärts-Primer, die Probenidentifizierungs-Sequenz der Rückwärts-Primer, die Untergruppen-Identifizierungs-Sequenz der weiteren Vorwärts-Primer bzw. die Untergruppen-Identifizierungs-Sequenz der weiteren Rückwärts-Primer einen Sequenzabstand umfassen, vorzugsweise einen Hamming-Abstand oder eine Levenshtein-Distanz, zu anderen Probenidentifizierungs-Sequenzen von Vorwärts-Primern, Probenidentifizierungs-Sequenzen von Rückwärts-Primern, Untergruppen-Identifizierungs-Sequenzen von weiteren Vorwärts-Primern bzw. Untergruppen-Identifizierungs-Sequenzen von weiteren Rückwärts-Primern, von mindestens 2, vorzugsweise von mindestens 3.

14. Verfahren nach einem der Ansprüche 1 bis 13, wobei die Probenidentifizierungs-Sequenz der Vorwärts-Primer, die Probenidentifizierungs-Sequenz der Rückwärts-Primer, die Untergruppen-Identifizierungs-Sequenz der weiteren Vorwärts-Primer bzw. die Untergruppen-Identifizierungs-Sequenz der weiteren Rückwärts-Primer, die jeweils unabhängig voneinander ausgewählt werden, eine Länge von mindestens 4 Nukleotiden aufweisen.

15. Ein Satz von Primern, geeignet für ein Verfahren nach einem der Ansprüche 1 bis 14, umfassend
mindestens 10 verschiedene Primer A, die von 5' bis 3' die Sequenz umfassen: eine Adapter-Sequenz A, eine Identifizierungs-Sequenz mit einer Länge von mindestens 4 nt und eine Ziel-Bindungssequenz, wobei die Identifizierungs-Sequenz innerhalb der mindestens 10 verschiedenen Primer A unterschiedlich ist, vorzugsweise mit einem Sequenzabstand von einem Hamming-Abstand von mindestens 1 oder einer Levenshtein-Distanz von mindestens 1, und wobei die Probenidentifizierungs-Sequenz und die Bindungssequenz für die Hybridisierung an die Analyten-Nukleinsäuren um einen zufälligen Versatz von 1-4 Basen versetzt sind; mindestens 10 verschiedene Primer B, die von 5' bis 3' die Sequenz umfassen: eine Adapter-Sequenz, eine Identifizierungs-Sequenz mit einer Länge von mindestens 4 nt und eine Ziel-Bindungssequenz, wobei die Identifizierungs-Sequenz innerhalb der mindestens 10 verschiedenen Primer B unterschiedlich ist, vorzugsweise mit einem Sequenzabstand von einem Hamming-Abstand von mindestens 1 oder einer Levenshtein-Distanz von mindestens 1;
mindestens 10 verschiedene Primer C, die von 5' bis 3' die Sequenz umfassen: eine Adapter-Sequenz C, eine Identifizierungs-Sequenz von mindestens 4 nt Länge und eine Bindungssequenz, die an die Adapter-Sequenz A bindet, wobei die Identifizierungs-Sequenz innerhalb der mindestens 10 verschiedenen Primer C unterschiedlich ist, vorzugsweise mit einem Sequenzabstand eines Hamming-Abstands von mindestens 1 oder einer Levenshtein-Distanz von mindestens 1; und
mindestens 10 verschiedene Primer D, die von 5' bis 3' die Sequenz umfassen: eine Adapter-Sequenz D, eine Identifizierungs-Sequenz von mindestens 4 nt Länge und eine Bindungssequenz, die an die Adapter-Sequenz B bindet, wobei die Identifizierungs-Sequenz innerhalb der mindestens 10 verschiedenen Primer D unterschiedlich ist, vorzugsweise mit einem Sequenzabstand eines Hamming-Abstands von mindestens 1 oder einer Levenshtein-Distanz von mindestens 1.

16. Satz von Primern nach Anspruch 15, wobei die Ziel-Bindungssequenzen der Primer A und Primer B eine Sequenz bzw. Antisense-Sequenz eines viralen Gens, vorzugsweise eines SARS-CoV-2 N-, M-, E- oder S-Gens oder einer kodierenden Sequenz davon, eines Influenza-PA-, PB1-, PB2-, PA-, HA-, NP-, NA-, Ml-, M2-, NS1- oder NEP-Gens oder einer kodierenden Sequenz davon, eines Parainfluenza-HN-, -F-, -M-, -NP-, -P- oder -L-Gens oder einer kodierenden Sequenz davon, eines Rhinovirus-VP1-, -VP2-, - VP3-, -VP4-, -2A-, -2B-, -2C-, 3A-, -3B-, -3C- oder -3D-Gens oder einer kodierenden Sequenz davon; eines humanen respiratorischen Synzytialvirus A (HRSV-A) GA1, GA2, GA3, GA4, GA5, GA6, GA7, SAA1, NA1 oder NA2 Gens oder einer kodierenden Sequenz ; eines humanen respiratorischen Synzytialvirus B (HRSV-B) GB1, GA2, GA3, GB4, SAB1, SAB2, SAB3, BA1, BA2, BA3, BA4, BA5 oder BA6 Gens oder kodierenden Sequenz umfassen.

17. Satz von Primern nach Anspruch 15 oder 16, ferner umfassend ein Primer-Paar, das an ein ribosomales Gen oder eine codierende Sequenz bindet.

## Revendications

1. Une méthode pour détecter un acide nucléique d'intérêt dans une pluralité d'échantillons, comprenant les étapes de :
a) fournir des acides nucléiques analytes à partir d'une pluralité d'échantillons dans des conteneurs séparés pour chaque échantillon, où les conteneurs sont disposés en un agencement de sous-ensembles, où le agencement comprend deux ou plusieurs sous-ensembles ;
b) amplifier les acides nucléiques par une réaction d'extension d'amorce en utilisant au moins une paire d'amorces hybridées aux acides nucléiques analytes, où une paire d'amorces comprend une amorce sens et une amorce antisens, où les amorces sens et antisens comprennent toutes deux dans la direction 5' à 3' une séquence d'adaptateur, une séquence d'identifiant d'échantillon et une séquence de liaison pour l'hybridation aux acides nucléiques analytes, respectivement, et où dans l'amorce sens, la séquence d'identifiant d'échantillon et la séquence de liaison pour l'hybridation aux acides nucléiques analytes sont décalées par un décalage aléatoire de 1-4 bases ;
c) combiner les acides nucléiques amplifiés de l'étape b) des conteneurs de deux ou plusieurs sous-ensembles en un ensemble de conteneurs combinés, où les conteneurs d'un sous-ensemble, mais pas d'un autre sous-ensemble, sont combinés en un conteneur combiné ;
d) amplifier les acides nucléiques par une réaction d'extension d'amorce en utilisant au moins une paire d'amorces supplémentaires hybridées aux acides nucléiques amplifiés des conteneurs combinés de l'étape c), où une paire d'amorces supplémentaires comprend une amorce sens supplémentaire et une amorce antisens supplémentaire, où les amorces sens supplémentaire et antisens supplémentaire comprennent toutes deux une séquence d'identifiant de sous-ensemble et une séquence pour l'hybridation à la séquence d'adaptateur ;
e) déterminer les séquences des acides nucléiques amplifiés de l'étape d) ;
f) attribuer une séquence déterminée d'un acide nucléique d'intérêt de l'étape e) à un échantillon par association à un sous-ensemble et conteneur avec les séquences d'identifiant de sous-ensemble et les séquences d'identifiant d'échantillon.

2. La méthode selon la revendication 1, où i) à l'étape b) chaque séquence d'identifiant d'échantillon des amorces sens est différente de la séquence d'identifiant d'échantillon des autres amorces sens pour une séquence de liaison donnée pour l'hybridation aux acides nucléiques analytes ; et/ou
ii) à l'étape b) chaque séquence d'identifiant d'échantillon des amorces antisens est différente de la séquence d'identifiant d'échantillon des autres amorces antisens pour une séquence de liaison donnée pour l'hybridation aux acides nucléiques analytes ;
et/ou
iii) à l'étape d) chaque séquence d'identifiant de sous-ensemble des amorces sens supplémentaires est différente de la séquence d'identifiant de sous-ensemble des autres amorces sens supplémentaires ;
et/ou
iv) à l'étape d) chaque séquence d'identifiant de sous-ensemble des amorces antisens supplémentaires est différente de la séquence d'identifiant de sous-ensemble des autres amorces antisens supplémentaires ;
de préférence une combinaison de i), ii), iii) et iv).

3. La méthode selon la revendication 1 ou 2, où les amorces supplémentaires de l'étape d) comprennent une séquence d'adaptateur de séquençage.

4. La méthode selon l'une quelconque des revendications 1 à 3, où l'étape a) comprend la fourniture d'un ARN à partir d'un échantillon et la génération d'un ADNc dudit ARN par transcription inverse, où les acides nucléiques qui sont amplifiés à l'étape b) comprennent ledit ADNc.

5. La méthode selon l'une quelconque des revendications 1 à 4, où au moins l'une des amorces sens et antisens de l'étape b) est épuisée pendant et/ou après l'étape b) mais avant l'étape c).

6. La méthode selon la revendication 5, où l'épuisement comprend l'amplification d'un acide nucléique de spike-in avec l'amorce sens et/ou antisens pendant l'étape b).

7. La méthode selon la revendication 5 ou 6, où l'épuisement des amorces sens et antisens comprend un traitement avec une nucléase spécifique à simple brin.

8. La méthode selon l'une quelconque des revendications 1 à 7, où l'amplification des acides nucléiques à l'étape d) est limitée à au plus 35 cycles d'amplification, de préférence au plus 20 cycles d'amplification.

9. La méthode selon l'une quelconque des revendications 1 à 8, où la pluralité d'échantillons comprend 4000 échantillons ou plus et/ou les deux ou plusieurs sous-ensembles sont 40 sous-ensembles ou plus et/ou où un sous-ensemble comprend 40 conteneurs ou plus.

10. La méthode selon l'une quelconque des revendications 1 à 9, où l'acide nucléique d'intérêt est un acide nucléique pathogène, de préférence un acide nucléique viral.

11. La méthode selon la revendication 10, où l'acide nucléique d'intérêt est sélectionné parmi un acide nucléique de coronavirus, de préférence un acide nucléique de SARS-CoV-2, un acide nucléique de grippe, un acide nucléique de parainfluenza, un acide nucléique de rhinovirus, ou une combinaison de ceux-ci.

12. La méthode selon l'une quelconque des revendications 1 à 11, où deux ou plusieurs acides nucléiques d'intérêt sont sélectionnés, de préférence où les deux ou plusieurs acides nucléiques d'intérêt proviennent d'un virus, qui est le même virus ou un virus différent pour deux ou plusieurs acides nucléiques d'intérêt, et/ou de préférence où à l'étape b) des amorces sens et antisens avec des séquences de liaison pour l'hybridation aux deux ou plusieurs acides nucléiques analytes sont utilisées.

13. La méthode selon l'une quelconque des revendications 1 à 12, où la séquence d'identifiant d'échantillon des amorces sens, la séquence d'identifiant d'échantillon des amorces antisens, la séquence d'identifiant de sous-ensemble des amorces sens supplémentaires, et la séquence d'identifiant de sous-ensemble des amorces antisens supplémentaires, respectivement, présentent une distance de séquence, de préférence une distance de Hamming ou une distance de Levenshtein, aux autres séquences d'identifiant d'échantillon des amorces sens, séquences d'identifiant d'échantillon des amorces antisens, séquences d'identifiant de sous-ensemble des amorces sens supplémentaires, et séquences d'identifiant de sous-ensemble des amorces antisens supplémentaires, respectivement, d'au moins 2, de préférence d'au moins 3.

14. La méthode selon l'une quelconque des revendications 1 à 13, où la séquence d'identifiant d'échantillon des amorces sens, la séquence d'identifiant d'échantillon des amorces antisens, la séquence d'identifiant de sous-ensemble des amorces sens supplémentaires, et la séquence d'identifiant de sous-ensemble des amorces antisens supplémentaires, respectivement, chacune sélectionnée indépendamment, ont une longueur d'au moins 4 nucléotides.

15. Un ensemble d'amorces adapté à une méthode selon l'une quelconque des revendications 1 à 14, comprenant :
au moins 10 amorces A différentes qui comprennent la séquence, de 5' à 3' : une séquence d'adaptateur A, une séquence d'identifiant d'au moins 4 nt de longueur et une séquence de liaison cible, où la séquence d'identifiant est différente dans les au moins 10 amorces A différentes, de préférence avec une distance de séquence d'une distance de Hamming d'au moins 1 ou une distance de Levenshtein d'au moins 1, et où la séquence d'identifiant d'échantillon et la séquence de liaison pour l'hybridation aux acides nucléiques analytes sont décalées par un décalage aléatoire de 1-4 bases ;
au moins 10 amorces B différentes qui comprennent la séquence, de 5' à 3' : une séquence d'adaptateur B, une séquence d'identifiant d'au moins 4 nt de longueur et une séquence de liaison cible, où la séquence d'identifiant est différente dans les au moins 10 amorces B différentes, de préférence avec une distance de séquence d'une distance de Hamming d'au moins 1 ou une distance de Levenshtein d'au moins 1 ;
au moins 10 amorces C différentes qui comprennent la séquence, de 5' à 3' : une séquence d'adaptateur C, une séquence d'identifiant d'au moins 4 nt de longueur et une séquence de liaison qui se lie à la séquence d'adaptateur A, où la séquence d'identifiant est différente dans les au moins 10 amorces C différentes, de préférence avec une distance de séquence d'une distance de Hamming d'au moins 1 ou une distance de Levenshtein d'au moins 1 ; et
au moins 10 amorces D différentes qui comprennent la séquence, de 5' à 3' : une séquence d'adaptateur D, une séquence d'identifiant d'au moins 4 nt de longueur et une séquence de liaison qui se lie à la séquence d'adaptateur B, où la séquence d'identifiant est différente dans les au moins 10 amorces D différentes, de préférence avec une distance de séquence d'une distance de Hamming d'au moins 1 ou une distance de Levenshtein d'au moins 1.

16. L'ensemble d'amorces selon la revendication 15, où les séquences de liaison cible des amorces A et amorces B comprennent une séquence ou une séquence antisens, respectivement, d'un gène viral, de préférence d'un gène N, M, E ou S du SARS-CoV-2 ou d'une séquence codante de celui-ci, d'un gène PA, PB1, PB2, PA, HA, NP, NA, M1, M2, NS1 ou NEP de la grippe ou d'une séquence codante de celui-ci, d'un gène HN, F, M, NP, P ou L de parainfluenza ou d'une séquence codante de celui-ci, d'un gène VP1, VP2, VP3, VP4, 2A, 2B, 2C, 3A, 3B, 3C ou 3D de rhinovirus ou d'une séquence codante ; d'un gène GA1, GA2, GA3, GA4, GA5, GA6, GA7, SAA1, NA1 ou NA2 du virus respiratoire syncytial humain A (HRSV-A) ou d'une séquence codante ; d'un gène GB1, GA2, GA3, GB4, SAB1, SAB2, SAB3, BA1, BA2, BA3, BA4, BA5 ou BA6 du virus respiratoire syncytial humain B (HRSV-B) ou d'une séquence codante.

17. L'ensemble d'amorces selon la revendication 15 ou 16, comprenant en outre une paire d'amorces qui se lie à un gène ribosomique ou à une séquence codante.
